(19) Europäisches Patentamt
European Patent Office
Office européen des brevets

(11) Veröffentlichungsnummer: **0 257 485 A2**

(12) **EUROPÄISCHE PATENTANMELDUNG**

(21) Anmeldenummer: **87111815.4**

(22) Anmeldetag: **14.08.87**

(51) Int. Cl.⁴: **A61K 31/44** , A61K 37/64 , //(A61K37/64,31:44)

(30) Priorität: **27.08.86 DE 3629060**

(43) Veröffentlichungstag der Anmeldung: **02.03.88 Patentblatt 88/09**

(84) Benannte Vertragsstaaten: **AT BE CH DE ES FR GB GR IT LI NL SE**

(71) Anmelder: **BAYER AG**
**Konzernverwaltung RP Patentabteilung**
**D-5090 Leverkusen 1 Bayerwerk(DE)**

(72) Erfinder: **Schramm, Matthias, Dr.**
**Paffrather Strasse 38**
**D-5000 Köln 80(DE)**
Erfinder: **Gross, Rainer, Dr.**
**Platzhofstrasse 23**
**D-5600 Wuppertal 1(DE)**
Erfinder: **Kayser, Michael, Dr.**
**Fleyerstrasse 231**
**D-5800 Hagen 1(DE)**

(54) **Kombination von positiv inotrop wirkenden Dihydropyridinen mit ACE-Hemmern, sowie ihre Verwendung in Arzneimitteln.**

(57) Die Erfindung betrifft eine Kombination von ACE-hemmenden Verbindungen der Formeln (I) und (II)

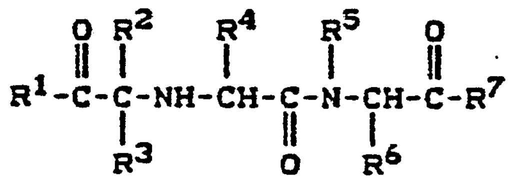

(I)

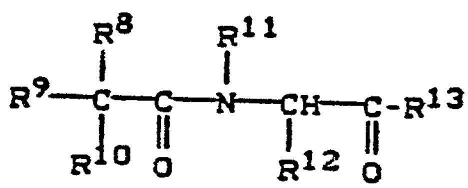

(II)

und von positiv-inotropen Dihydropyridinen der Formel (III)

EP 0 257 485 A2

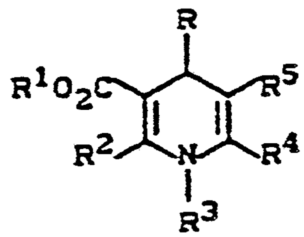

(III)

mit den in der Beschreibung angegebenen Substituentendefinitionen,
sowie ihre Verwendung in Arzneimitteln, insbesondere in blutdrucksenkenden Arzneimitteln.

## Kombination von positiv inotrop wirkenden Dihydropyridinen mit ACE-Hemmern, sowie ihre Verwendung in Arzneimitteln

Die vorliegende Erfindung betrifft eine Kombination von positiv-inotrop wirkenden Dihydropyridinverbindungen (Komponente B) mit Verbindungen, die die Bildung von Enzymen hemmen, welche die Umwandlung von Angiotensin I in Angiotensin II steuern (Komponente A), sowie ihre Verwendung in Arzneimitteln, insbesondere in blutdrucksenkenden Arzneimitteln.

Verbindungen, die die Bildung von Enzymen hemmen die Angiotensin I in Angiotensin II umwandeln, werden als ACE-Hemmer bezeichnet (Angiotensin Converting Enzyme). Diese Verbindungen sind als blutdrucksenkende Mittel bekannt und werden neben der Behandlung des Bluthochdrucks auch zur Behandlung der Herzinsuffizienz eingesetzt, um die Nachlast des Herzens zu senken.

ACE-hemmende Verbindungen müssen zur Behandlung der Herzinsuffizienz im allgemeinen mit Glykosiden kombiniert werden, da sie nicht in der Lage sind, die Kontraktionskraft des Herzens zu steigern.

Als ACE-hemmende Verbindungen, seien diejenigen der Formeln (I) und (II) genannt

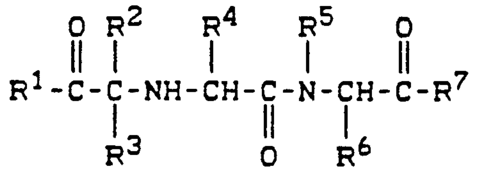

(I)

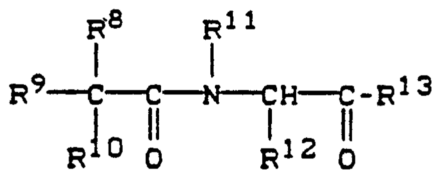

(II)

in welchen

$R^1$,$R^7$ -gleich oder verschieden sind und

-für Hydroxy, Alkoxy mit bis zu 10 Kohlenstoffatomen, Aryloxy mit 6 bis 12 Kohlenstoffatomen oder Aralkoxy mit 7 bis 14 Kohlenstoffatomen stehen,

$R^2$ -für Wasserstoff oder

-für Alkyl mit bis zu 4 Kohlenstoffatomen steht,

$R^3$ -für Wasserstoff oder

-für eine Gruppe der Formel

$-(CH_2)_m-R^{14}$ steht,

worin

m = eine Zahl 1,2,3,4,5 oder 6 bedeutet

und

$R^{14}$ -Wasserstoff, Cycloalkyl mit 3 bis 8 Kohlenstoffatomen, Hydroxy, Carboxy, Alkoxycarbonyl mit bis zu 4 Kohlenstoffatomen, Amino, Guanidino, Alkylamino mit bis zu 6 Kohlenstoffatomen, Dialkylamino mit bis zu 6 Kohlenstoffatomen je Alkylgruppe, einen 5-bis 7-gliedrigen Heterocyclus mit Sauerstoff, Schwefel oder Stickstoff als Heteroatome, oder Aryl mit 6 bis 12 Kohlenstoffatomen bedeutet, wobei dieser Arylrest durch Halogen, Cyano, Nitro, Alkyl oder Alkoxy mit jeweils bis zu 4 Kohlenstoffatomen substituiert sein kann,

oder

$R^2$ und $R^3$ gemeinsam eine Gruppe der Formel

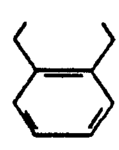

bilden,

$R^4$ -für Wasserstoff oder
-für eine Gruppe der Formel
$-(CH_2)_n-R^{15}$ steht,
worin
n - die gleiche Bedeutung hat wie m und mit diesem gleich oder verschieden sein kann
und
$R^{15}$ -die gleiche Bedeutung hat wie $R^{14}$ und mit diesem gleich oder verschieden sein kann,
$R^5$ -für Wasserstoff oder
-für Alkyl mit bis zu 8 Kohlenstoffatomen steht, das substituiert sein kann durch eine Gruppe der Formel

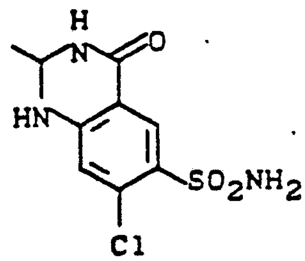

oder
-für Cycloalkyl mit 3 bis 8 Kohlenstoffatomen steht, an das ein Phenylring anelliert sein kann, oder
-für eine Gruppe $-NR^{16}R^{17}$ steht,
worin
$R^{16}$,$R^{17}$ -gleich oder verschieden sind und Wasserstoff, Alkyl mit bis zu 6 Kohlenstoffatomen, Aryl mit 6 bis 12 Kohlenstoffatomen, Aralkyl mit 7 bis 14 Kohlenstoffatomen oder Acyl mit 2 bis 7 Kohlenstoffatomen bedeuten,
$R^4$ und $R^5$ -gemeinsam eine Gruppe der Formel

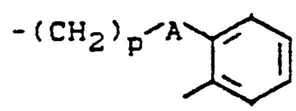

bilden, worin
p - eine Zahl 1,2,3 oder 4 bedeutet
und
A - eine direkte Bindung, Sauerstoff, Schwefel, NH, N-Alkyl mit bis zu 6 Kohlen stoffatomen, N-Phenyl, -CHCOOH oder -CHCOO-Alkyl mit bis zu 6 Kohlenstoffatomen in der Alkylkette bedeutet,
$R^6$ -für Wasserstoff steht,
oder
$R^6$ und $R^5$ gemeinsam eine Gruppe der Formel

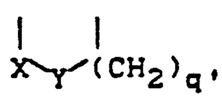 , 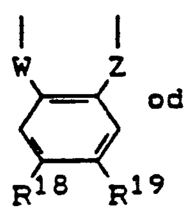 oder 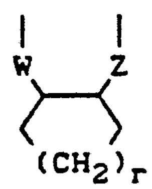 bilden,

worin
X - eine Methylen-oder Carbonylgruppe bedeutet,
Y - Schwefel, NH, N-Alkyl mit bis zu 6 Kohlenstoffatomen in der Alkylgruppe, N-Phenyl, eine Carbonylgruppe oder eine Gruppe $-CHR^{20}$ bedeutet,
wobei

$R^{20}$ -für Hydroxy, Mercapto, Phenyloxy, Phenylthio, Alkoxy oder Alkylthio mit jeweils bis zu 6 Kohlenstoffatomen je Alkylgruppe steht,

W,Z - gleich oder verschieden sind und eine direkte Bindung oder eine Methylen-oder Ethylengruppe bedeuten,

q,r - unabhängig voneinander eine Zahl 1,2,3 oder 4 bedeuten,

$R^{18}$,$R^{19}$ gleich oder verschieden sind und Wasserstoff, Alkyl oder Alkoxy mit jeweils bis zu 6 Kohlenstoffatomen bedeuten,

oder die Gruppierung

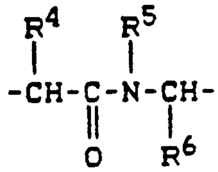

in Formel (I) für eine Gruppierung der Formel

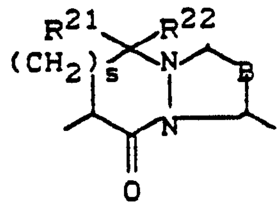

steht, worin

s - eine Zahl 0,1,2 oder 3 bedeutet,

B - eine Methylen-, Ethylen-oder Vinylengruppe bedeutet

und

$R^{21}$,$R^{22}$ -jeweils Wasserstoff oder Alkyl mit bis zu 4 Kohlenstoffatomen oder zusammen eine Oxogruppe bedeuten,

$R^8$ -für Wasserstoff oder

-für Alkyl mit bis zu 6 Kohlenstoffatomen steht,

$R^9$ -für Wasserstoff oder

- für einen Rest der Formel

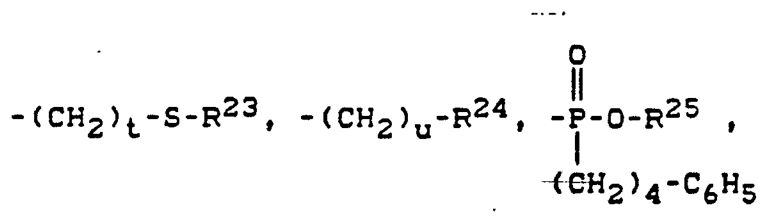

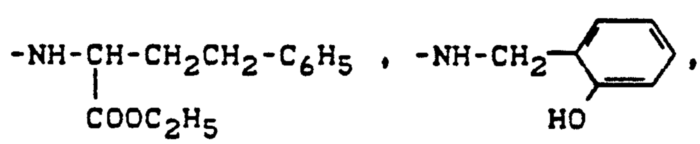

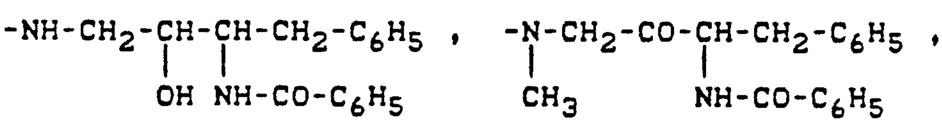

-NH-CS-NH-$C_6H_5$ oder 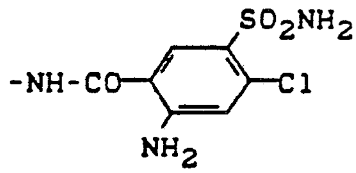 steht,

worin
t - eine Zahl 0,1,2,3 oder 4 bedeutet,
u - eine Zahl 0,1,2,3 oder 4 bedeutet,
$R^{23}$ -Wasserstoff, Alkyl, Alkylthio oder Alkylcarbonyl mit jeweils bis zu 6 Kohlenstoffatomen im Alkylteil, Benzoyl oder eine Gruppe der Formel

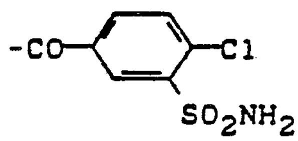 , 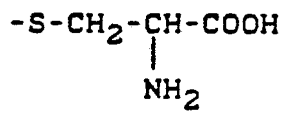 ,

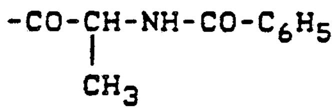 oder

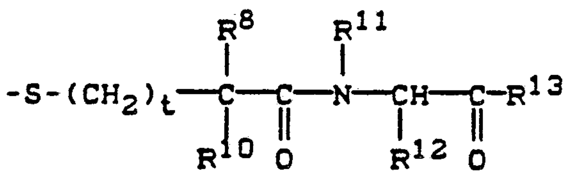 bedeutet,

$R^{24}$ -Wasserstoff, Alkyl oder Alkylcarbonyl mit bis zu 8 Kohlenstoffatomen im Alkylteil, Carboxy, Alkoxycarbonyl mit bis zu 6 Kohlenstoffatomen oder eine Gruppe der Formel

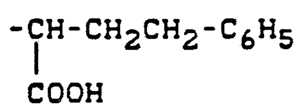 , 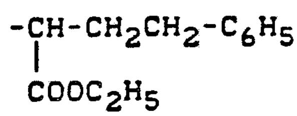 ,

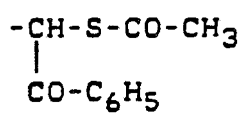 , 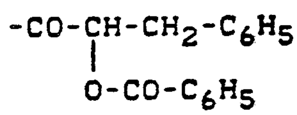

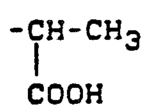 oder 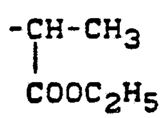 bedeutet

und
$R^{25}$ -Wasserstoff oder eine Gruppe der Formel

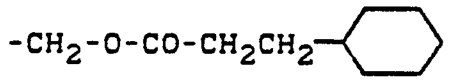,

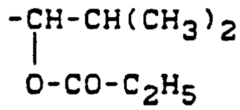 oder 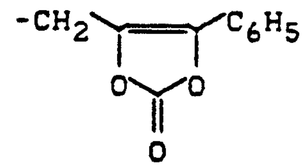

bedeutet,
$R^{10}$ -für Wasserstoff oder Alkyl mit bis zu 6 Kohlenstoffatomen steht,
$R^{11}$ -für Wasserstoff oder für Alkyl mit bis zu 8 Kohlenstoffatomen steht, das substituiert sein kann durch einen 5-bis 7-gliedrigen Heterocyclus mit Sauerstoff, Schwefel oder Stickstoff als Heteroatomen, oder -für Cycloalkyl mit 3 bis 7 Kohlenstoffatomen steht, das durch Phenyl oder Alkyl mit bis zu 4 Kohlenstoffatomen substituiert sein kann,
oder
$R^{10}$ und $R^{11}$ gemeinsam eine Gruppe der Formel

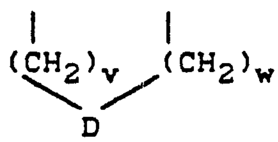

bilden,
worin
v - eine Zahl 0,1,2,3 oder 4 bedeutet,
w - eine Zahl 0,1,2 oder 3 bedeutet
und
D - eine direkte Bindung, Sauerstoff, Schwefel, NH oder eine ortho-Phenylen-Gruppe bedeutet,
$R^{12}$ -für Wasserstoff oder für Alkyl mit bis zu 6 Kohlenstoffatomen steht,das durch eine Indolylgruppe substituiert sein kann,
$R^{11}$ und $R^{12}$ gemeinsam eine Gruppe der Formel

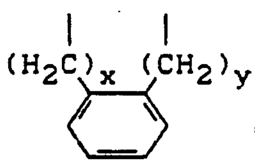 oder 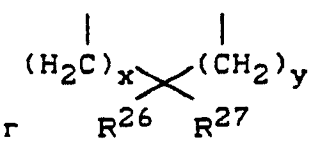

bilden,
worin
x,y - gleich oder verschieden sind und eine Zahl 0,1,2,3 oder 4 bedeuten,
$R^{26}$ -Wasserstoff bedeutet,
$R^{27}$ -Wasserstoff, Alkyl mit bis zu 6 Kohlenstoffatomen, Cycloalkyl mit 3 bis 7 Kohlenstoffatomen, Phenylthio, Phenylsulfonyl, Alkylthio oder Alkylsulfonyl mit jeweils bis zu 4 Kohlenstoffatomen, oder einen Rest der Formel

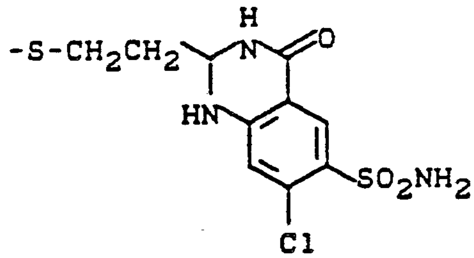

oder

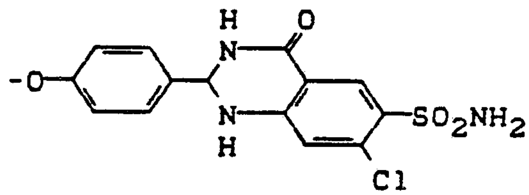

bedeutet,
oder
$R^{26}$ und $R^{27}$ gemeinsam eine Gruppe der Formel

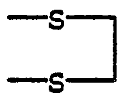 , 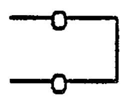 oder 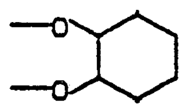 bilden

und
$R^{13}$ -für Hydroxy oder Alkoxy mit bis zu 6 Kohlenstoffatomen steht,
und deren physiologisch unbedenklichen Salze.

Bevorzugt seien Verbindungen der allgemeinen Formeln (I) und (II) genannt

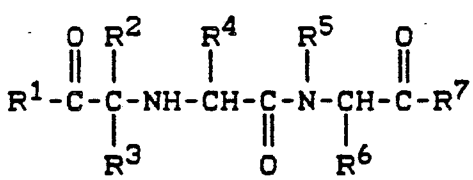 (I)

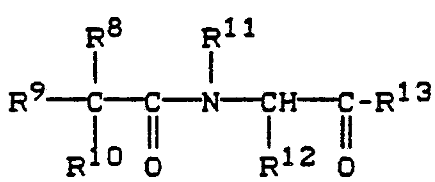 (II)

in welchen
$R^1$,$R^7$ -gleich oder verschieden sind und
-für Hydroxy, Alkoxy mit bis zu 6 Kohlenstoffatomen, Phenyloxy oder Benzyloxy stehen,
$R^2$ -für Wasserstoff oder für Alkyl mit bis zu 4 Kohlenstoffatomen steht,
$R^3$ -für Wasserstoff oder für eine Gruppe der Formel
$-(CH_2)_m-R^{14}$ steht,
worin
m - eine Zahl 1,2,3 oder 4 bedeutet

und

$R^{14}$ -Wasserstoff, Cycloalkyl mit 3 bis 6 Kohlenstoffatomen, Phenyl, Amino, Acetylamino, Alkylamino oder Dialkylamino mit jeweils bis zu 4 Kohlenstoffatomen je Alkylgruppe, Piperidin, Piperazin, Morpholin oder Thiomorpholin bedeutet,

oder

$R^2$ und $R^3$ -gemeinsam eine Gruppe der Formel

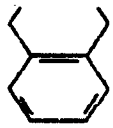

bilden,

$R^4$ - für Wasserstoff oder eine Gruppe der Formel

$-(CH_2)_n-R^{15}$ steht,

worin

n - die gleiche Bedeutung hat wie m und mit diesem gleich oder verschieden sein kann,

und

$R^{15}$ -die gleiche Bedeutung hat wie $R^{14}$ und mit diesem gleich oder verschieden sein kann

$R^5$ -für Wasserstoff oder für Alkyl mit bis zu 4 Kohlenstoffatomen steht, das substituiert sein kann durch eine Gruppe der Formel

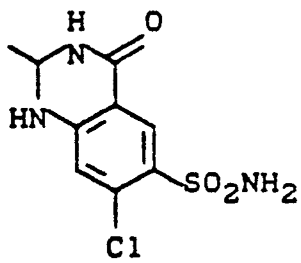

oder

-für Cyclopentyl oder Cyclohexyl steht, an die auch ein Phenylring anelliert sein kann, oder

-für eine Gruppe $-NR^{16}R^{17}$ steht,

worin

$R^{16},R^{17}$ -gleich oder verschieden sind und Wasserstoff, Alkyl mit bis zu 3 Kohlenstoffatomen, Phenyl, Benzyl oder Acetyl bedeuten,

oder

$R^4$ und $R^5$ -gemeinsam ein Gruppe der Formel

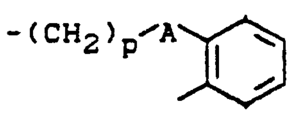

bilden,

worin

p - eine Zahl 1,2 oder 3 bedeutet

und

A - eine direkte Bindung, Sauerstoff, Schwefel, NH, N-Phenyl, N-Methyl, -CHCOOH oder -CHCOO-Alkyl mit bis zu 4 Kohlenstoffatomen in der Alkylkette bedeutet,

$R^6$ -für Wasserstoff steht,

oder

$R^5$ und $R^6$ -gemeinsam eine Gruppe der Formel

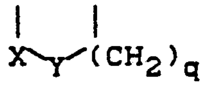, 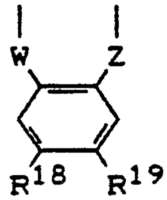 oder 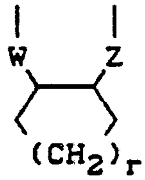 bilden,

worin
X - eine Methylen-oder eine Carbonylgruppe bedeutet,
Y - Sauerstoff, NH, N-Phenyl, N-Alkyl mit bis zu 4 Kohlenstoffatomen, eine Carbonylgruppe oder eine Gruppe $-CHR^{20}$ bedeutet,
wobei
$R^{20}$ -für Mercapto, Alkylthio mit bis zu 4 Kohlenstoffatomen,
-für Phenylthio, oder für Alkoxycarbonyl mit bis zu 4 Kohlenstoffatomen steht,
W,Z - gleich oder verschieden sind und eine direkte Bindung oder eine Methylengruppe bedeuten,
q,r - unabhängig voneinander eine Zahl 1,2 oder 3 bedeuten
und
$R^{18}$,$R^{19}$ -gleich oder verschieden sind und Wasserstoff oder Alkoxy mit bis zu 4 Kohlenstoffatomen bedeuten,
oder die Gruppierung

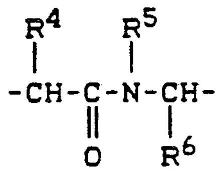

in Formel (I) für eine Gruppierung der Formel

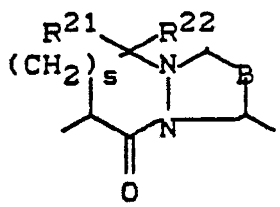

steht,
worin
s - eine Zahl 0,1 oder 2 bedeutet,
B - eine Methylen-oder Ethylengruppe bedeutet
und
$R^{21}$,$R^{22}$ -jeweils Wasserstoff oder zusammen eine Oxogruppe bedeuten
$R^{8}$ -für Wasserstoff oder Alkyl mit bis zu 4 Kohlenstoffatomen steht,
$R^{9}$ -für Wasserstoff oder
-für einen Rest der Formel

$-(CH_2)_t-S-R^{23}$, $-(CH_2)_u-R^{24}$, 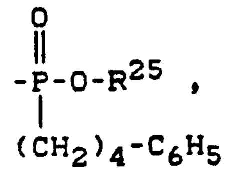 ,

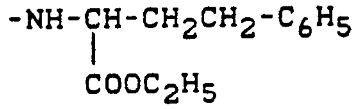 , 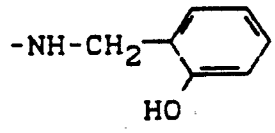 ,

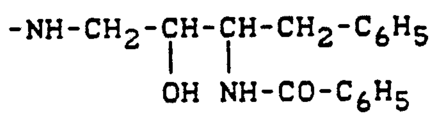 , 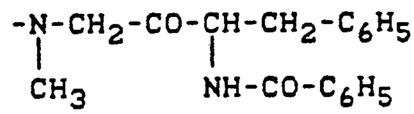 ,

$-NH-CS-NH-C_6H_5$, 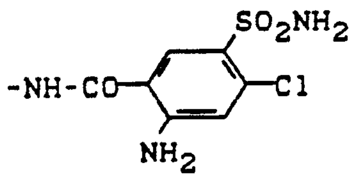 steht,

worin

t - eine Zahl 0,1,2 oder 3 bedeutet,

u - eine Zahl 0,1,2 oder 3 bedeutet,

$R^{23}$ -Wasserstoff, Alkyl, Alkylthio oder Alkylcarbonyl mit jeweils bis zu 4 Kohlenstoffatomen je Alkylgruppe, Benzoyl oder eine Gruppe der Formel

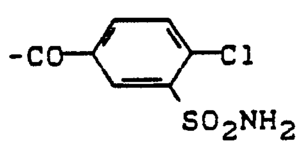 , 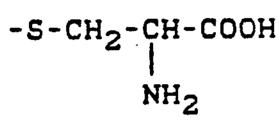 ,

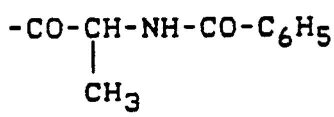 oder

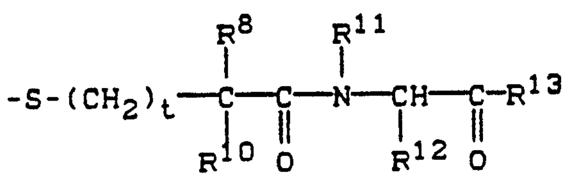 bedeutet,

$R^{24}$ -Wasserstoff, Alkyl, Alkylcarbonyl mit jeweils bis zu 6 Kohlenstoffatomen im Alkylteil, Carboxy, Alkoxycarbonyl mit bis zu 4 Kohlenstoffatomen oder eine Gruppe der Formel

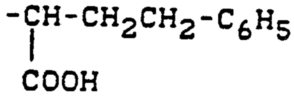 , 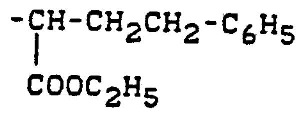 ,

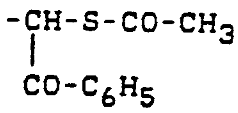 , 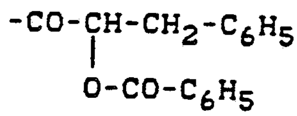

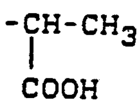 oder 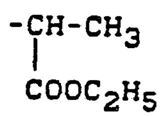 bedeutet

und

$R^{25}$ -Wasserstoff oder eine Gruppe der Formel

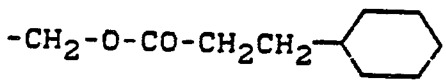 ,

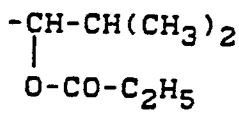 oder 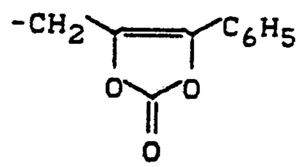

bedeutet,

$R^{10}$ -für Wasserstoff oder Alkyl mit bis zu 4 Kohlenstoffatomen steht,

$R^{11}$ -für Wasserstoff oder für Alkyl mit bis zu 4-Kohlenstoffatomen steht, das substituiert sein kann durch einen 5-bis 6-gliedrigen Heterocyclus mit Sauerstoff, Schwefel oder Stickstoff als Heteroatomen,oder für Cyclopentyl oder Cyclohexyl steht, das durch Phenyl substituiert sein kann,

oder

$R^{10}$ und $R^{11}$ -gemeinsam eine Gruppe der Formel

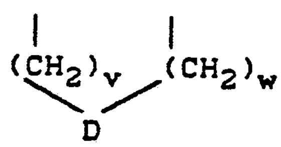

bilden,

worin

v - eine Zahl 0,1,2 oder 3 bedeutet,

2 - eine Zahl 0,1 oder 2 bedeutet

und

D - eine direkte Bindung, Sauerstoff, Schwefel oder eine ortho-Phenylen-Gruppe bedeutet,

$R^{12}$ -für Wasserstoff oder für Alkyl mit bis zu 4 Kohlenstoffatomen steht, das durch eine Indolylgruppe substituiert sein kann,

oder

$R^{11}$ und $R^{12}$ -gemeinsam eine Gruppe der Formel

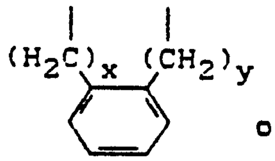 oder 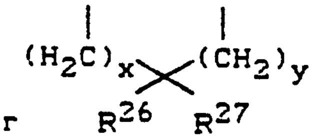 bilden,

worin

x,y - gleich oder verschieden sind und eine Zahl 0,1,2 oder 3 bedeuten,

$R^{26}$ - Wasserstoff bedeutet,

$R^{27}$ -Wasserstoff, Alkyl mit bis zu 4 Kohlenstoffatomen, Cyclopentyl, Cyclohexyl, Phenylthio, Phenylsulfonyl, Methylthio, Methylsulfonyl oder einen Rest der Formel

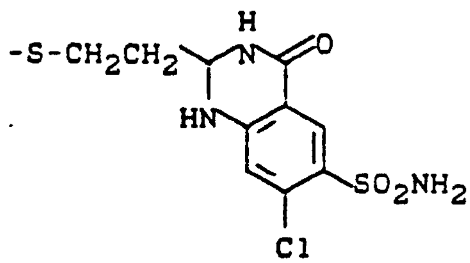

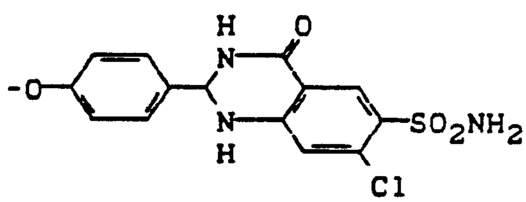

bedeutet,

oder

$R^{26}$ und $R^{27}$ gemeinsam eine Gruppe der Formel

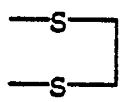 , 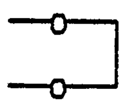 oder 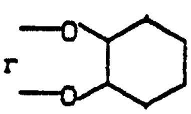 bilden

und

$R^{13}$ -für Hydroxy oder Alkoxy mit bis zu 4 Kohlenstoffatomen steht

und deren physiologisch unbedenklichen Salze.

Besonders bevorzugt seien Verbindungen der Formel (I) und (II) genannt,

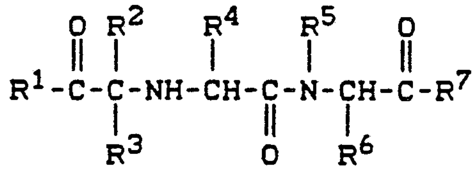

(I)

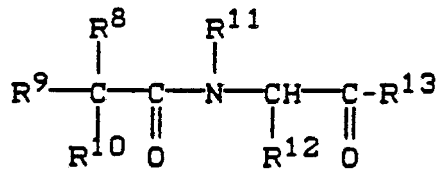

(II)

worin
$R^1$ -für Hydroxy oder Ethoxy steht,
$R^2$ -für Wasserstoff steht,
$R^3$ -für Propyl, Benzyl, 2-Phenylethyl, 2-Cyclohexylethyl, 2-(4-Piperidinyl)ethyl oder 4-Aminobutyl steht,
oder
$R^2$ und $R^3$ -gemeinsam eine Gruppe der Formel

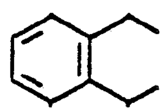

bilden,
$R^4$ -für Wasserstoff, Methyl oder 4-Aminobutyl steht,
$R^5$ -für Wasserstoff oder eine Gruppe der Formel

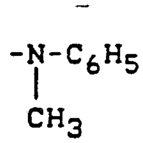

,

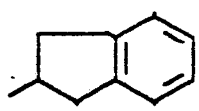

oder

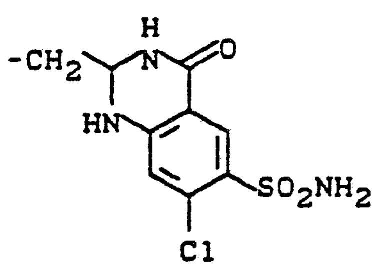

steht,

oder
$R^4$ und $R^5$ -gemeinsam eine Gruppe der Formel

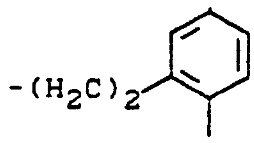 , 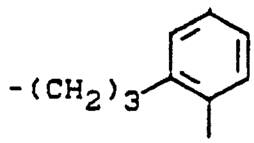 , 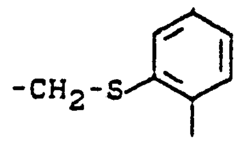

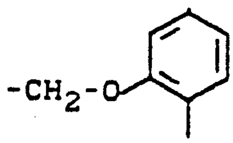 oder 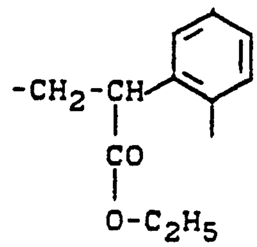 bilden ,

$R^6$ -für Wasserstoff steht,
oder
$R^5$ und $R^6$ -gemeinsam eine Gruppe der Formel

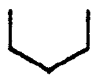 , 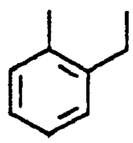 , 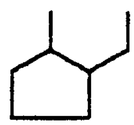 , 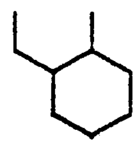 ,

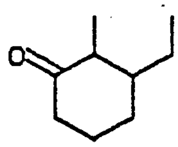 , 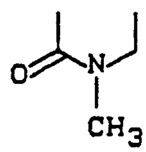 , 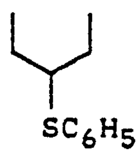 , 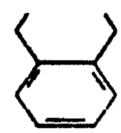 oder

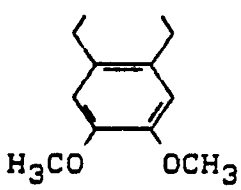 bilden,

oder die Gruppierung

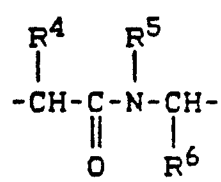

in Formel (I) für die Gruppierung der Formel

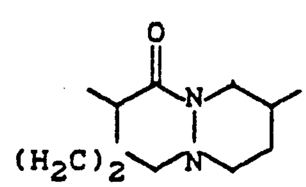

steht,
$R^7$ -für Hydroxy steht,
$R^8$ -für Wasserstoff oder Methyl steht,
$R^9$ -für eine Gruppe der Reihe

$-SH$, $-CH_2SH$, $-CH_2-S-CO-C_6H_5$, 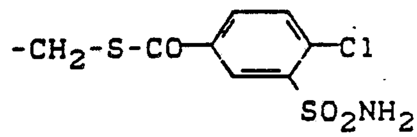

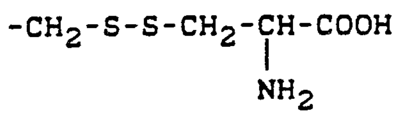 , 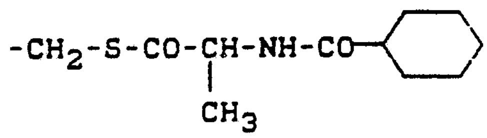

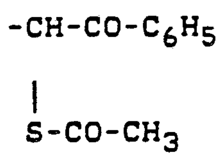 , $-CH_2-CH_2-COOH$, 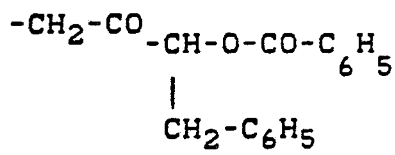

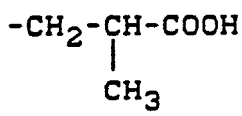 , 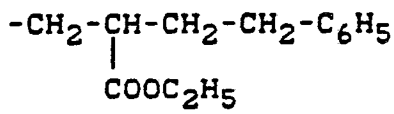

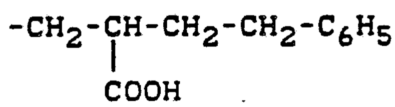 , 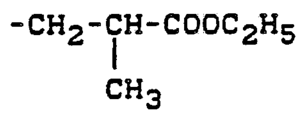

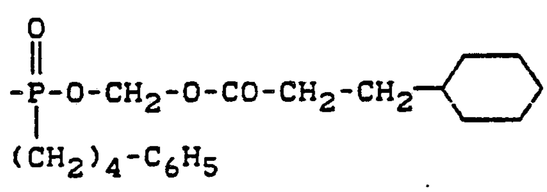 , 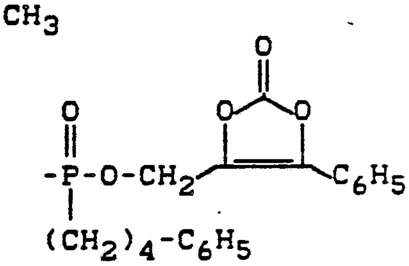

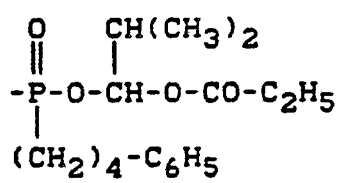 , 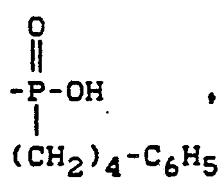 ,

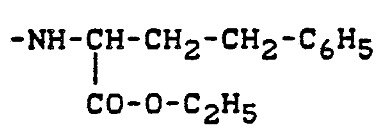 , 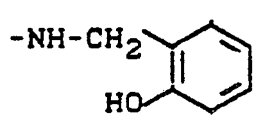 ,

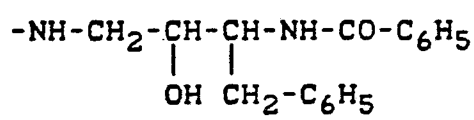 ,

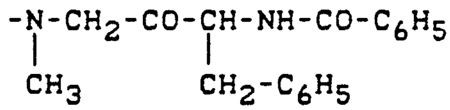,

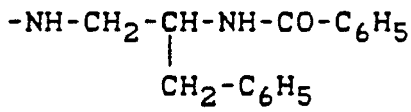,

$-NH-CS-NH-C_6H_5$, 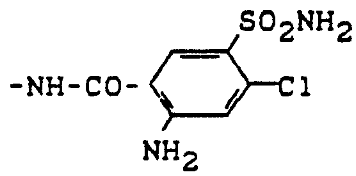 oder

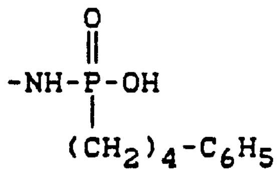 steht,

$R^{10}$ -für Wasserstoff steht,
$R^{11}$ -für Wasserstoff oder für eine Gruppe der Formel

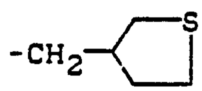 oder 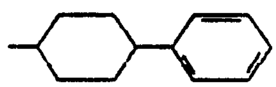 steht,

oder
$R^{10}$ und $R^{11}$ -gemeinsam eine Gruppe der Formel

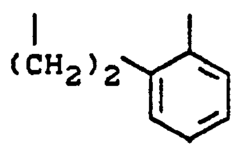, 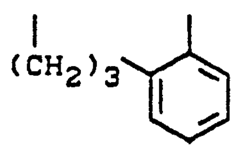, 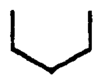,

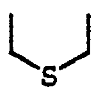 oder 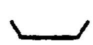 bilden,

$R^{12}$ -für Wasserstoff, Methyl oder Indol-3-yl-methyl steht,
oder
$R^{11}$ und $R^{12}$ -gemeinsam eine Gruppe der Formel

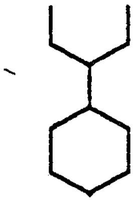, 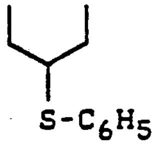, 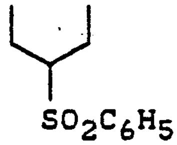, 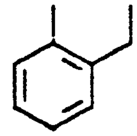,

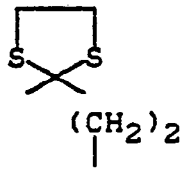, 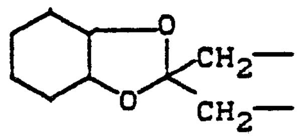

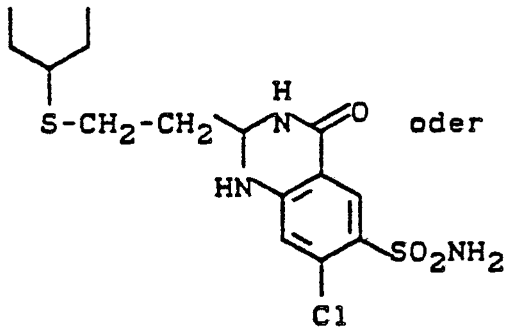 oder 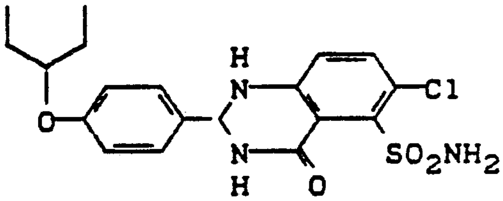

bilden
und
$R^{13}$ -für Hydroxy steht
und deren physiologisch unbedenkliche Salze.

Die Herstellung dieser Verbindungen ist beschrieben in: Europäische Offenlegungsschriften 12 401, 37 231, 49 505, 50 850, 79 022, 49 095; Deutsche Offenlegungsschrift 27 03 828; US-Patentschrift 4.316.906.

Positiv-inotrope Dihydropyridine sind in der Lage die Kontraktionskraft des Herzmuskels zu steigern, wobei diese Wirkung durch eine Steigerung des Calciumeinstromes in die Muskelzelle eintritt.

Als positiv-inotrope Dihydropyridine seien diejenigen der folgenden Formel (II) genannt

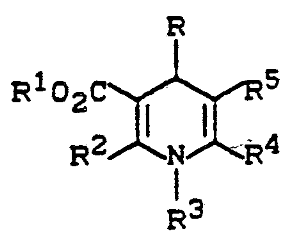 (III)

in welcher
R - für Cycloalkyl mit 3 bis 14 Kohlenstoffatomen,
-für Aryl mit 6 bis 14 Kohlenstoffatomen oder für Heteroaryl steht, die substituiert sein können durch bis zu 5 gleiche oder verschiedene Substituenten der Reihe: Halogen, Nitro, Cyano, Trifluormethyl, Monofluoralkoxy mit bis zu 12 Kohlenstoffatomen, Polyfluoralkoxy mit bis zu 12 Kohlenstoffatomen, Hydroxy, Amino, Alkylamino mit bis zu 8 Kohlenstoff atomen, Dialkylamino mit bis zu 8 Kohlenstoffatomen je Alkylgruppe, Aryl mit 6 bis 12 Kohlenstoffatomen, Heteroaryl, Aralkyl mit 7 bis 14 Kohlenstoffatomen, Aralkoxy mit 7 bis 14 Kohlenstoffatomen oder -$SO_n$-Aralkyl mit 7-14 Kohlenstoffatomen und n = 0-2, wobei die Substituenten der fünf letzt genannten Gruppen ebenfalls mehrfach substituiert sein können durch bis zu 5 Substituenten

der Reihe: Halogen, Nitro, Azido, Hydroxy, Trifluormethyl, Trifluormethoxy, Cyano, Amino, Alkylamino mit bis zu 8 Kohlenstoffatomen, Dialkylamino mit jeweils bis zu 8 Kohlenstoffatomen je Alkylgruppe, Alkoxy oder Alkylthio mit jeweils bis zu 4 Kohlenstoffatomen,

$R^1$ -für einen geradkettigen, verzweigten oder cyclischen, gesättigten oder ungesättigten Kohlenwasserstoffrest mit bis zu 20 Kohlenstoffatomen steht, der bis zu 5 Schwefel und/oder Sauerstoffatome in der Kette unterbrochen sein kann und der substituiert sein kann durch Halogen, Nitro, Hydroxy, Cyano, Trialkylsilyl mit jeweils bis zu 8 Kohlenstoffatomen pro Alkylgruppe, Alkoxycarbonyl mit bis zu 4 Kohlenstoffatomen, Amino, Alkylamino mit bis zu 4 Kohlenstoffatomen oder Dialkylamino mit jeweils bis zu 4 Kohlenstoffatomen je Alkylgruppe,

$R^2$,$R^4$ -gleich oder verschieden sind und

-für Wasserstoff, Amino, Cyano, Formyl oder

-für einen geradkettigen, verzweigten oder cyclischen, gesättigten oder ungesättigten Kohlenwasserstoffrest mit bis zu 10 Kohlenstoffatomen stehen, der substituiert sein kann durch Hydroxy, Carboxy, Alkoxycarbonyl mit bis zu 4 Kohlenstoffatomen oder durch Halogen,

$R^3$ -für Wasserstoff oder

-für einen geradkettigen, verzweigten oder cyclischen, gesättigten oder ungesättigten Kohlenwasserstoffrest mit bis zu 10 Kohlenstoffatomen steht, der substituiert sein kann durch Halogen, Cyano, Hydroxy, Amino, Alkylamino, Dialkylamino mit jeweils bis zu 4 Kohlenstoffatomen je Alkylgruppe oder durch einen 5-bis 7-gliedrigen heterocyclischen Ring, der als Heteroatome Stickstoff und/oder Sauerstoff und/oder Schwefel enthalten kann und sowohl gesättigt als auch ungesättigt sein kann

und

$R^5$ -für Wasserstoff,

-für Cyano oder

-für Nitro steht,

oder

$R^4$ und $R^5$ -gemeinsam einen Ring bilden wie

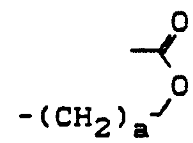 , 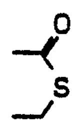 oder 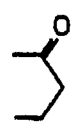 ,

wobei

a - für 1 oder 2 steht.

Bevorzugt seien Verbindungen der allgemeinen Formel (III) genannt

in welcher

R - für Cycloalkyl mit 4 bis 12 Kohlenstoffatomen oder

-für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei die genannten Reste substituiert sein können durch bis zu 4 gleiche oder verschiedene Substituenten der Reihe: Fluor, Chlor, Brom, Iod, Nitro, Cyano, Trifluormethyl, Monofluoralkoxy mit bis zu 8 Kohlenstoffatomen, Polyfluoralkoxy mit bis zu 8 Kohlenstoffatomen, Hydroxy, Amino, Alkylamino mit bis zu 6 Kohlenstoffatomen, Dialkylamino mit bis zu 6 Kohlenstoffatomen je Alkylgruppe, Phenyl, Naphthyl, Thienyl, Furyl, Pyridyl, Pyrimidyl, Benzyl, Benzyloxy, Benzylsulfonyl, wobei die Aromaten und Heterocyclen wiederum ein-bis vierfach substituiert sein können durch Fluor, Chlor, Brom, Iod, Cyano, Nitro, Azido, Hydroxy, Trifluormethyl, Trifluormethoxy, Amino, Alkylamino mit bis zu 6 Kohlenstoffatomen, Dialkylamino mit bis zu 6 Kohlenstoffatomen je Alkylgruppe Alkoxy oder Alkylthio mit jeweils bis zu 2 Kohlenstoffatomen,

$R^1$ -einen geradkettigen, verzweigten oder cyclischen, gesättigten oder ungesättigten Kohlenwasserstoffrest mit bis zu 17 Kohlenstoffatomen steht, der in der Kette durch bis zu 4 Sauerstoff-und/oder Schwefelatome unterbrochen sein kann und der substituiert sein kann durch ein oder mehrere Fluor, Chlor, Brom, Iod, Nitro, Hydroxy, Cyano, Trialkylsilyl mit jeweils bis zu 6 Kohlenstoffatomen je Alkylgruppe, Alkoxycarbonyl mit bis zu 2 Kohlenstoffatomen, Amino, Alkylamino oder Dialkylamino mit jeweils bis zu 2 Kohlenstoffatomen je Alkylgruppe,

$R^2$,$R^4$ -gleich oder verschieden sind und

-für Wasserstoff, Amino, Cyano, Formyl oder
-für einen geradkettigen, verzweigten oder cyclischen, gesättigten oder ungesättigten Kohlenwasserstoffrest mit bis zu 8 Kohlenstoffatomen stehen, der durch Hydroxy, Carboxy, Alkoxycarbonyl mit bis zu 2 Kohlenstoffatomen ein oder mehreren Fluor, Chlor oder durch Brom substituiert sein kann,
$R^3$ -für Wasserstoff oder
-für einen geradkettigen, verzweigten oder cyclischen, gesättigten oder ungesättigten Kohlenwasserstoffrest mit bis zu 8 Kohlenstoffatomen steht, der durch bis zu 3 Sauerstoffatome in der Kette unterbrochen sein kann und der substituiert sein kann durch ein oder mehrere Fluor, Chlor, Brom, Iod, Cyano, Hydroxy, Amino, Alkylamino mit bis zu 2 Kohlenstoffatomen, Dialkylamino mit jeweils bis zu 2 Kohlenstoffatomen pro Alkylgruppe, Morpholin, Piperidin, Pyridazin oder Pyridin
und
$R^5$ -für Wasserstoff, Cyano oder Nitro steht,
oder
$R^4$ und $R^5$ -gemeinsam einen Ring bilden wie

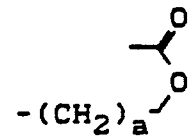 , 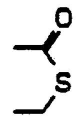 oder 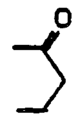 ,

wobei
a - für 1 oder 2 steht.

Besonders bevorzugt seien Verbindungen der allgemeinen Formel (III) genannt, in welcher
R - für Cycloalkyl mit 5 bis 10 Kohlenstoffatomen steht, oder
-für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinazolyl, Chinoxalyl, Thionaphthenyl, Isothionaphthenyl, Chromonyl, Thiochromonyl, Chromenyl, Thiochromenyl, Benzoxadiazolyl oder Benzothiadiazolyl steht, wobei die genannten Reste substituiert sein können durch bis zu 3 gleiche oder verschiedene Substituenten der Reihe: Fluor, Chlor, Brom, Nitro, Cyano, Trifluormethyl, Monofluoralkoxy mit bis zu 4 Kohlenstoffatomen, Polyfluoralkoxy mit bis zu 4 Kohlenstoffatomen, Hydroxy, Amino, Alkylamino, Dialkylamino mit jeweils bis zu 4 Kohlenstoffatomen pro Alkylgruppe, Phenyl, Thienyl, Pyridyl, Benzyl, Benzyloxy oder Benzylsulfonyl, wobei die Aromaten und Heteroaromaten wiederum ein-bis dreifach substituiert sein können durch Fluor, Chlor, Brom, Cyano, Nitro, Hydroxy, Trifluormethyl, Trifluormethoxy, Amino, Alkylamino mit bis zu 4 Kohlenstoffatomen, Dialkylamino mit jeweils bis zu 4 Kohlenstoffatomen je Alkylgruppe, Methoxy oder Methylthio,
$R^1$ -für einen geradkettigen, verzweigten oder cyclischen gesättigten oder ungesättigten Kohlenwasserstoffrest mit bis zu 14 Kohlenstoffatomen steht, der durch bis zu 3 Sauerstoff-und/oder Schwefelatome in der Kette unterbrochen sein kann und der substituiert sein kann durch ein oder mehrere Fluor, Chlor, Brom, Nitro, Hydroxy, Cyano oder Trialkylsilyl mit jeweils bis zu 4 Kohlenstoffatomen je Alkylgruppe,
$R^2$,$R^4$ -gleich oder verschieden sind und
-für Wasserstoff, Amino, Cyano, Formyl oder
-für einen geradkettigen oder verzweigten, gesättigten oder ungesättigten Kohlenwasserstoffrest mit bis zu 6 Kohlenstoffatomen stehen, der durch Hydroxy substituiert sein kann,
$R^3$ -für Wasserstoff oder
-für einen geradkettigen, verzweigten oder cyclischen, gesättigten oder ungesättigten Kohlenwasserstoffrest mit bis zu 6 Kohlenstoffatomen steht, der durch bis zu 2 Sauerstoffatome in der Kette unterbrochen sein kann und der substituiert sein kann durch ein oder mehrere Fluor, Chlor, Cyano, Hydroxy, Amino oder Morpholino
und
$R^5$ -für Wasserstoff,
-für Cyano oder
-für Nitro steht,
oder
$R^4$ und $R^5$ -gemeinsam einen Ring bilden wie

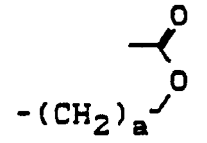 , 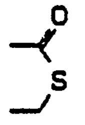 oder 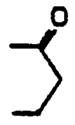 ,

wobei

a - für 1 oder 2 steht.

Insbesondere seien genannt:

1,4-Dihydro-2,6-dimethyl-5-nitro-4-(2-trifluormethylphenyl)pyridin-3-carbonsäuremethylester 2-Methyl-4-(4-oxo-2-phenyl-4H-thiochromen-8-yl)-5-oxo-1,4,5,7-tetrahydrofuro[3,4-b]pyridin-3-ethylester.

Die Herstellung dieser Verbindungen ist in den Deutschen Offenlegungsschriften 32 06 671, 33 11 005 und 33 11 003 beschrieben.

Die vorliegende Erfindung beruht auf der Feststellung, daß durch Kombination mit positiv-inotropen Dihydropyridinen die Nachlast senkende Wirkung der ACE-Hemmer nicht aufgehoben wird, und daß zusätzlich die positiv inotrope Wirkung der Dihydropyridine voll zur Geltung kommt. Mit der vorliegenden Kombination wird daher ein wesentlich größerer Patientenkreis behandelbar, als es mit den Einzelkomponenten möglich ist.

Bezogen auf 1 Gewichtsteil eines ACE-Hemmers können 0,1 bis 10 Gewichtsteile, bevorzugt 0,1 bis 5 Gewichtsteile, des Dihydropyridins eingesetzt werden.

Die Kombination kann hergestellt werden, indem man die Einzelkomponenten in einem diese auflösenden inerten Lösemittel auflöst und gegebenenfalls nach Abdampfen des Lösemittels die Kombination in üblicher Weise mit Hilfsstoffen vermischt. Als inerte Lösemittel seien beispielhaft Alkohole wie Ethanol, oder Glykole wie Polyethylenglykol genannt.

Wie bereits erwähnt kann die erfindungsgemäße Kombination zur Bekämpfung von Erkrankungen, besonders von Herz-und Kreislauf-Erkrankungen, insbesondere zur Behandlung der Herzinsuffizienz verwendet werden.

Die neue Wirkstoffkombination kann in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht toxischer, pharmazeutisch geeigneter Trägerstoffe oder Lösungsmittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffkombination mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuß/Sesamöl), Alkohole (z. B. Ethylalkohol, Glycerin), Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch-und Traubenzucker), Emulgiermittel (z.B. Polyoxyethylen-Fettsäure-Ester), Polyoxyethylen-Fettalkohol-Ether (z.B. Lignin, Sulfitablaugen, Methylcellulose, Stärke und Polyvinylpyrrolidon) und Gleitmittel (z.B. Magnesiumstearat, Talkum, Stearinsäure und Natriumsulfat).

Die Applikation erfolgt in üblicher Weise, vorzugsweise oral oder parenteral, beispielsweise intravenös. Im Falle der oralen Anwendung können Tabletten selbstverständlich außer den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wäßriger Suspensionen kann die Wirkstoffkombination außer den obengenannten Hilfsstoffen mit verschiedenen Geschmacksaufbesserern oder Farbstoffen versetzt werden.

Für den Fall der parenteralen Anwendung können Lösungen der Wirkstoffkombination unter Verwendung geeigneter flüssiger Trägermaterialien eingesetzt werden.

Herstellungsbeispiele

Beispiel 1

Zunächst werden 100 mg 1,4-Dihydro-2,6-dimethyl-5-nitro-4-(2-trifluormethylphenyl)pyridin-3-carbonsäuremethylester in 100 ml Adalat-Placebo-Lösung (60 g Glycerin, 100 g $H_2O$, PEG 400 ad 1129 g) gelöst, dann werden 100 mg Captopril in 100 ml Adalat-Placebo-Lösung gelöst und anschließend die beiden Lösungen im Verhältnis 1:1 gemischt.

Beispiel 2

Zunächst werden 100 mg 1,4-Dihydro-2,6-dimethyl-5-nitro-4-(2-trifluormethylphenyl)pyridin-3-carbonsäuremethylester in 100 ml Adalat-Placebo-Lösung (60 g Glycerin, 100 g $H_2O$, PEG 400 ad 1129 g) gelöst, dann werden 100 mg Enalapril in 100 ml Adalat-Placebo-Lösung gelöst und anschließend die beiden Lösungen im Verhältnis 1:1 gemischt.

**Ansprüche**

1. Pharmazeutisches Kombinationspräparat enthaltend mindestens einen Wirkstoff aus der Gruppe ACE-Hemmer (Komponente A) der allgmemeinen Formeln I und II

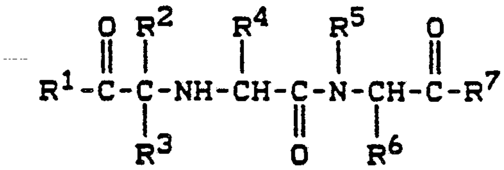 (I)

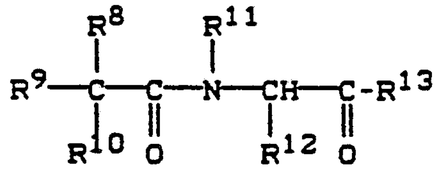 (II)

in welchen
$R^1$,$R^7$ -gleich oder verschieden sind und
-für Hydroxy, Alkoxy mit bis zu 10 Kohlenstoffatomen, Aryloxy mit 6 bis 12 Kohlenstoffatomen oder Aralkoxy mit 7 bis 14 Kohlenstoffatomen stehen,
$R^2$ -für Wasserstoff oder
-für Alkyl mit bis zu 4 Kohlenstoffatomen steht,
$R^3$ -für Wasserstoff oder
-für eine Gruppe der Formel
$-(CH_2)_m-R^{14}$ steht,
worin
m - eine Zahl 1,2,3,4,5 oder 6 bedeutet
und
$R^{14}$ -Wasserstoff, Cycloalkyl mit 3 bis 8 Kohlenstoffatomen, Hydroxy, Carboxy, Alkoxycarbonyl mit bis zu 4 Kohlenstoffatomen, Amino, Guanidino, Alkylamino mit bis zu 6 Kohlenstoffatomen, Dialkylamino mit bis zu 6 Kohlenstoffatomen je Alkylgruppe, einen 5-bis 7-gliedrigen Heterocyclus mit Sauerstoff, Schwefel oder Stickstoff als Heteroatome, oder Aryl mit 6 bis 12 Kohlenstoffatomen bedeutet, wobei dieser Arylrest durch Halogen, Cyano, Nitro, Alkyl oder Alkoxy mit jeweils bis zu 4 Kohlenstoffatomen substituiert sein kann,
oder
$R^2$ und $R^3$gemeinsam eine Gruppe der Formel

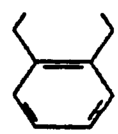

bilden,
$R^4$ -für Wasserstoff oder
-für eine Gruppe der Formel
$-(CH_2)_n-R^{15}$steht,
worin
n - die gleiche Bedeutung hat wie m und mit diesem gleich oder verschieden sein kann
und
$R^{15}$ -die gleiche Bedeutung hat wie $R^{14}$ und mit diesem gleich oder verschieden sein kann,
$R^5$ -für Wasserstoff oder
-für Alkyl mit bis zu 8 Kohlenstoffatomen steht, das substituiert sein kann durch eine Gruppe der Formel

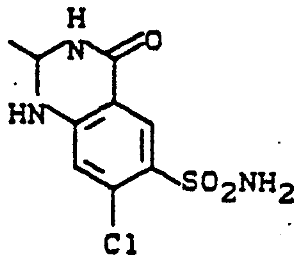

oder
-für Cycloalkyl mit 3 bis 8 Kohlenstoffatomen steht, an das ein Phenylring anelliert sein kann, oder
-für eine Gruppe $-NR^{16}R^{17}$ steht,
worin
$R^{16}$,$R^{17}$ -gleich oder verschieden sind und Wasserstoff, Alkyl mit bis zu 6 Kohlenstoffatomen, Aryl mit 6 bis 12 Kohlenstoffatomen, Aralkyl mit 7 bis 14 Kohlenstoffatomen oder Acyl mit 2 bis 7 Kohlenstoffatomen bedeuten,
$R^4$ und $R^5$-gemeinsam eine Gruppe der Formel

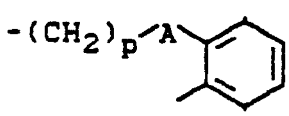

bilden,
worin
p - eine Zahl 1,2,3 oder 4 bedeutet
und
A - eine direkte Bindung oder Sauerstoff, Schwefel, NH, N-Alkyl mit bis zu 6 Kohlenstoffatomen, N-Phenyl,
-CHCOOH oder
-CHCOO-Alkyl mit bis zu 6 Kohlenstoffatomen in der Alkylkette bedeutet,
$R^6$ -für Wasserstoff steht,
oder
$R^6$ und $R^5$ gemeinsam eine Gruppe der Formel

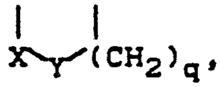 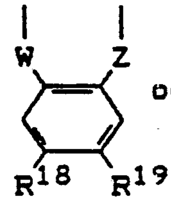 oder 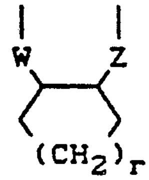 bilden,

worin

X - eine Methylen-oder Carbonylgruppe bedeutet,

Y - Schwefel, NH, N-Alkyl mit bis zu 6 Kohlenstoffatomen in der Alkylgruppe, N-Phenyl, eine Carbonylgruppe oder eine Gruppe -$CHR^{20}$ bedeutet,

wobei

$R^{20}$ -für Hydroxy, Mercapto, Phenyloxy, Phenylthio, Alkoxy oder Alkylthio mit jeweils bis zu 6 Kohlenstoffatomen je Alkylgruppe steht,

W,Z - gleich oder verschieden sind und eine direkte Bindung oder eine Methylen-oder Ethylengruppe bedeuten,

q,r - unabhängig voneinander eine Zahl 1,2,3 oder 4 bedeuten,

$R^{18}$,$R^{19}$ gleich oder verschieden sind und Wasserstoff, Alkyl oder Alkoxy mit jeweils bis zu 6 Kohlenstoffatomen bedeuten,

oder die Gruppierung

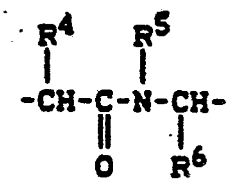

in Formel (I) für eine Gruppierung der Formel

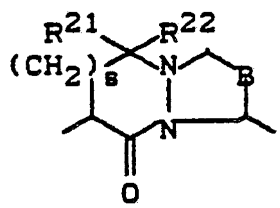

steht,

worin

s - eine Zahl 0,1,2 oder 3 bedeutet,

B - eine Methylen-, Ethylen-oder Vinylengruppe bedeutet

und

$R^{21}$,$R^{22}$ -jeweils Wasserstoff oder Alkyl mit bis zu 4 Kohlenstoffatomen oder zusammen eine Oxogruppe bedeuten,

$R^8$ -für Wasserstoff oder

-für Alkyl mit bis zu 6 Kohlenstoffatomen steht,

$R^9$ -für Wasserstoff oder

-für einen Rest der Formel

$-(CH_2)_t-S-R^{23}$, $-(CH_2)_u-R^{24}$, 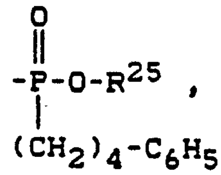 ,

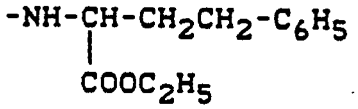 , 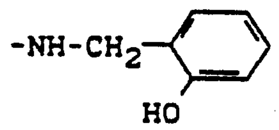 ,

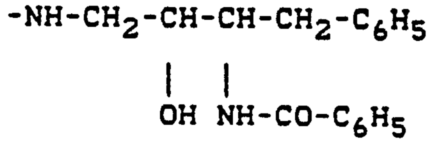 , 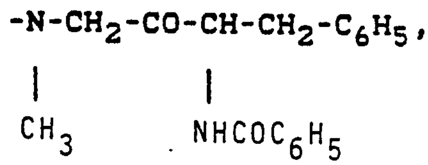 ,

$-NH-CS-NH-C_6H_5$ oder 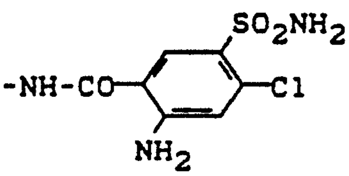 **steht,**

worin

t - eine Zahl 0,1,2,3 oder 4 bedeutet,

u - eine Zahl 0,1,2,3 oder 4 bedeutet,

$R^{23}$ -Wasserstoff, Alkyl, Alkylthio oder Alkylcarbonyl mit jeweils bis zu 6 Kohlenstoffatomen im Alkylteil, Benzoyl oder eine Gruppe der Formel

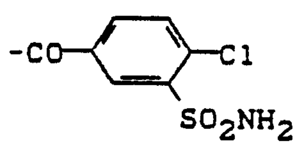 , 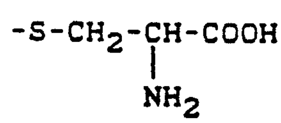 ,

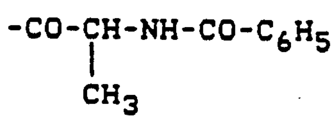 **oder**

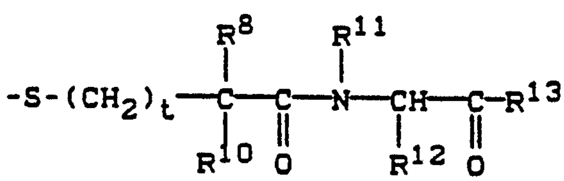 **bedeutet,**

$R^{24}$ -Wasserstoff, Alkyl oder Alkylcarbonyl mit bis zu 8 Kohlenstoffatomen im Alkylteil, Carboxy, Alkoxycarbonyl mit bis zu 6 Kohlenstoffatomen oder eine Gruppe der Formel

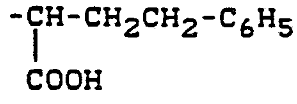 , 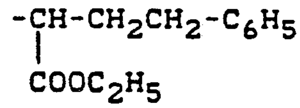 ,

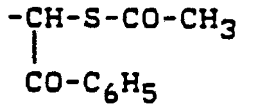 , 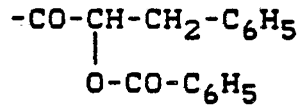

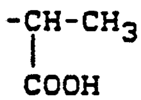 oder 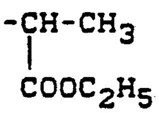 bedeutet

und

$R^{25}$ -Wasserstoff oder eine Gruppe der Formel

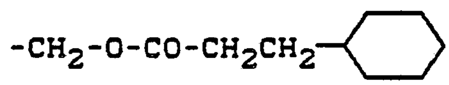 ,

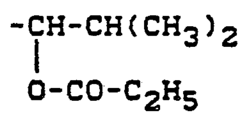 oder 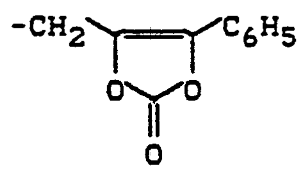

bedeutet,

$R^{10}$ -für Wasserstoff oder Alkyl mit bis zu 6 Kohlenstoffatomen steht,

$R^{11}$ -für Wasserstoff oder für Alkyl mit bis zu 8 Kohlenstoffatomen steht, das substituiert sein kann durch einen 5-bis 7-gliedrigen Heterocyclus mit Sauerstoff, Schwefel oder Stickstoff als Heteroatomen, oder -für Cycloalkyl mit 3 bis 7 Kohlenstoffatomen steht, das durch Phenyl oder Alkyl mit bis zu 4 Kohlenstoffatomen substituiert sein kann,

oder

$R^{10}$ und $R^{11}$ gemeinsam eine Gruppe der Formel

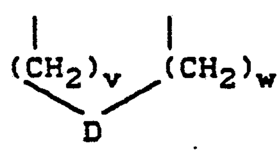 bilden,

worin

v - eine Zahl 0,1,2,3 oder 4 bedeutet,

w - eine Zahl 0,1,2 oder 3 bedeutet

und

D - eine direkte Bindung, Sauerstoff, Schwefel, NH oder eine ortho-Phenylen-Gruppe bedeutet,

$R^{12}$ -für Wasserstoff oder für Alkyl mit bis zu 6 Kohlenstoffatomen steht,das durch eine Indolylgruppe substituiert sein kann,

oder

$R^{11}$ und $R^{12}$ gemeinsam eine Gruppe der Formel

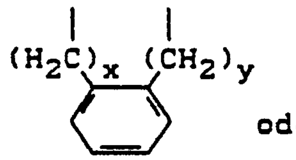 oder 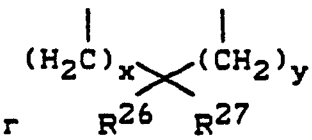 bilden,

worin

x,y - gleich oder verschieden sind und eine Zahl 0,1,2,3 oder 4 bedeuten,

$R^{26}$ -Wasserstoff bedeutet,

$R^{27}$ -Wasserstoff, Alkyl mit bis zu 6 Kohlenstoffatomen, Cycloalkyl mit 3 bis 7 Kohlenstoffatomen, Phenylthio, Phenylsulfonyl, Alkylthio oder Alkylsulfonyl mit jeweils bis zu 4 Kohlenstoffatomen, oder einen Rest der Formel

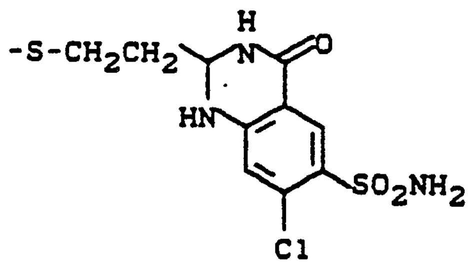 oder

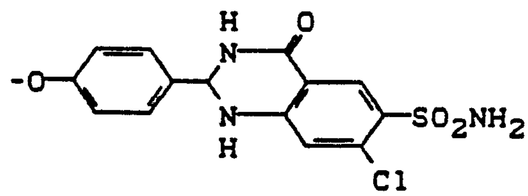

bedeutet,

oder

$R^{26}$ und $R^{27}$ gemeinsam eine Gruppe der Formel

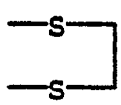, 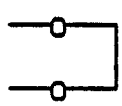 oder 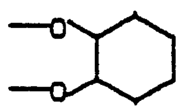 bilden

und

$R^{13}$ -für Hydroxy oder Alkoxy mit bis zu 6 Kohlenstoffatomen steht,

und deren physiologisch unbedenklichen Salze, sowie positiv inotrop wirkende Dihydropyridinverbindungen (Komponente B) der allgemeinen Formel III

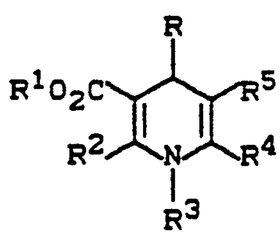 (III)

in welcher

R - für Cycloalkyl mit 3 bis 14 Kohlenstoffatomen,
-für Aryl mit 6 bis 14 Kohlenstoffatomen oder für Heteroaryl steht, die substituiert sein können durch bis zu 5 gleiche oder verschiedene Substituenten der Reihe: Halogen, Nitro, Cyano, Trifluormethyl, Monofluoralkoxy mit bis zu 12 Kohlenstoffatomen, Polyfluoralkoxy mit bis zu 12 Kohlenstoffatomen, Hydroxy, Amino, Alkylamino mit bis zu 8 Kohlenstoffatomen, Dialkylamino mit bis zu 8 Kohlenstoffatomen je Alkylgruppe, Aryl mit 6 bis 12 Kohlenstoffatomen, Heteroaryl, Aralkyl mit 7 bis 14 Kohlenstoffatomen, Aralkoxy mit 7 bis 14 Kohlenstoffatomen oder $-SO_n$ -Aralkyl mit 7 bis 14 Kohlenstoffatomen und n = 0-2, wobei die Substituenten der fünf letztgenannten Gruppen ebenfalls mehrfach substituiert sein können durch bis zu 5 Substituenten der Reihe: Halogen, Nitro, Azido, Hydroxy, Trifluormethyl, Trifluormethoxy, Cyano, Amino, Alkylamino mit bis zu 8 Kohlenstoffatomen , Dialkylamino mit jeweils bis zu 8 Kohlenstoffatomen je Alkylgruppe, Alkoxy oder Alkylthio mit jeweils bis zu 4 Kohlenstoffatomen,
$R^1$ -für einen geradkettigen, verzweigten oder cyclischen, gesättigten oder ungesättigten Kohlenwasserstoffrest mit bis zu 20 Kohlenstoffatomen steht, der bis zu 5 Schwefel und/oder Sauerstoffatome in der Kette unterbrochen sein kann und der substituiert sein kann durch Halogen, Nitro, Hydroxy, Cyano, Trialkylsilyl mit jeweils bis zu 8 Kohlenstoffatomen pro Alkylgruppe, Alkoxycarbonyl mit bis zu 4 Kohlenstoffatomen, Amino, Alkylamino mit bis zu 4 Kohlenstoffatomen oder Dialkylamino mit jeweils bis zu 4 Kohlenstoffatomen je Alkylgruppe,
$R^2$,$R^4$ -gleich oder verschieden sind und
-für Wasserstoff, Amino, Cyano, Formyl oder
-für einen geradkettigen, verzweigten oder cyclischen, gesättigten oder ungesättigten Kohlenwasserstoffrest mit bis zu 10 Kohlenstoffatomen stehen, der substituiert sein kann durch Hydroxy, Carboxy, Alkoxycarbonyl mit bis zu 4 Kohlenstoffatomen oder durch Halogen,
$R^3$ -für Wasserstoff oder
-für einen geradkettigen, verzweigten oder cyclischen, gesättigten oder ungesättigten Kohlenwasserstoffrest mit bis zu 10 Kohlenstoffatomen steht, der substituiert sein kann durch Halogen, Cyano, Hydroxy, Amino, Alkylamino, Dialkylamino mit jeweils bis zu 4 Kohlenstoffatomen je Alkylgruppe oder durch einen 5-bis 7-gliedrigen heterocyclischen Ring, der als Heteroatome Stickstoff und/oder Sauerstoff und/oder Schwefel enthalten kann und sowohl gesättigt als auch ungesättigt sein kann
und
$R^5$ -für Wasserstoff,
-für Cyano oder
-für Nitro steht,
oder
$R^4$ und $R^5$ -gemeinsam einen Ring bilden wie

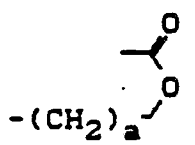
,
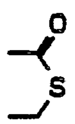
oder
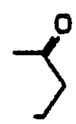

wobei
a - für 1 oder 2 steht.

2. Kombinationspräparat gemäß Anspruch 1 enthaltend mindestens eine Verbindung der allgemeinen Formeln I und II

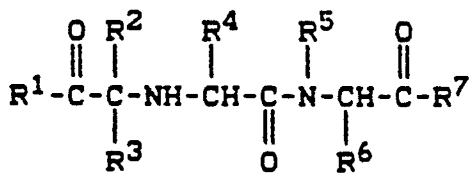

(I)

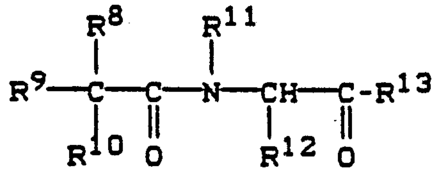

(II)

in welchen

$R^1$,$R^7$ -gleich oder verschieden sind und - für Hydroxy, Alkoxy mit bis zu 6 Kohlenstoffatomen, Phenyloxy oder Benzyloxy stehen,

$R^2$ -für Wasserstoff oder für Alkyl mit bis zu 4 Kohlenstoffatomen steht,

$R^3$ -für Wasserstoff oder für eine Gruppe der Formel

$-(CH_2)_m-R^{14}$ steht,

worin

m - eine Zahl 1,2,3 oder 4 bedeutet

und

$R^{14}$ -Wasserstoff, Cycloalkyl mit 3 bis 6 Kohlenstoffatomen, Phenyl, Amino, Acetylamino, Alkylamino oder Dialkylamino mit jeweils bis zu 4 Kohlenstoffatomen je Alkylgruppe, Piperidin, Piperazin, Morpholin oder Thiomorpholin bedeutet,

oder

$R^2$ und $R^3$ -gemeinsam eine Gruppe der Formel

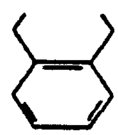

bilden,

$R^4$ - für Wasserstoff oder eine Gruppe der Formel

$-(CH_2)_n-R^{15}$ steht,

worin

n - die gleiche Bedeutung hat wie m und mit diesem gleich oder verschieden sein kann,

und

$R^{15}$ -die gleiche Bedeutung hat wie $R^{14}$ und mit diesem gleich oder verschieden sein kann,

$R^5$ -für Wasserstoff oder für Alkyl mit bis zu 4 Kohlenstoffatomen steht, das substituiert sein kann durch eine Gruppe der Formel

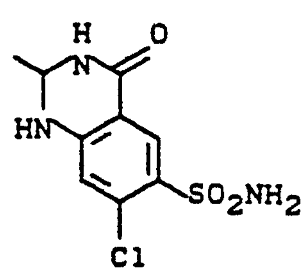

oder - für Cyclopentyl oder Cyclohexyl steht, an die auch ein Phenylring anelliert sein kann, oder - für eine Gruppe $-NR^{16}R^{17}$ steht,

worin
$R^{16}$,$R^{17}$ -gleich oder verschieden sind und Wasserstoff, Alkyl mit bis zu 3 Kohlenstoffatomen, Phenyl, Benzyl oder Acetyl bedeuten,
oder
$R^4$ und $R^5$-gemeinsam ein Gruppe der Formel

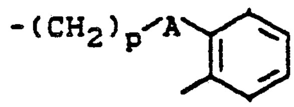

bilden,
worin
p - eine Zahl 1,2 oder 3 bedeutet
und
A - eine direkte Bindung, Sauerstoff, Schwefel, NH, N-Phenyl, N-Methyl, -CHCOOH oder -CHCOO-Alkyl mit bis zu 4 Kohlenstoffatomen in der Alkylkette bedeutet,
$R^6$ -für Wasserstoff steht,
oder
$R^5$ und $R^6$ -gemeinsam eine Gruppe der Formel

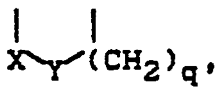 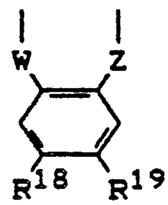 oder 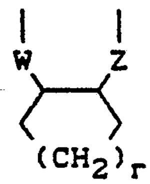

bilden
worin
X - eine Methylen-oder eine Carbonylgruppe bedeutet,
Y - Sauerstoff, NH, N-Phenyl, N-Alkyl mit bis zu 4 Kohlenstoffatomen, eine Carbonylgruppe oder eine Gruppe -$CHR^{20}$ bedeutet,
wobei
$R^{20}$ -für Mercapto, Alkylthio mit bis zu 4 Kohlenstoffatomen,
-für Phenylthio, oder für Alkoxycarbonyl mit bis zu 4 Kohlenstoffatomen steht,
W,Z - gleich oder verschieden sind und eine direkte Bindung oder eine Methylengruppe bedeuten,
q,r - unabhängig voneinander eine Zahl 1,2 oder 3 bedeuten
und
$R^{18}$,$R^{19}$ -gleich oder verschieden sind und Wasserstoff oder Alkoxy mit bis zu 4 Kohlenstoffatomen bedeuten,
oder die Gruppierung

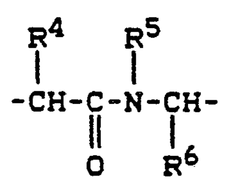

in Formel (I) für eine Gruppierung der Formel

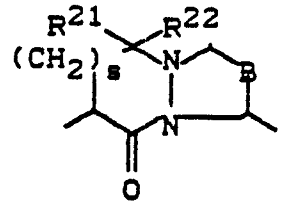

steht, worin
s - eine Zahl 0,1 oder 2 bedeutet,
B - eine Methylen-oder Ethylengruppe bedeutet
und
$R^{21}$,$R^{22}$ -jeweils Wasserstoff oder zusammen eine Oxogruppe bedeuten,
$R^8$ -für Wasserstoff oder Alkyl mit bis zu 4 Kohlenstoffatomen steht,
$R^9$ -für Wasserstoff oder
-für einen Rest der Formel

$-(CH_2)_t-S-R^{23}$, $-(CH_2)_u-R^{24}$, 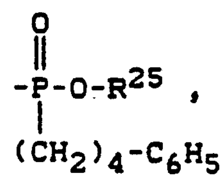 ,

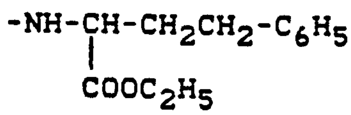 , 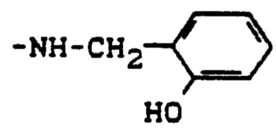 ,

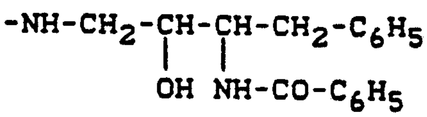 , 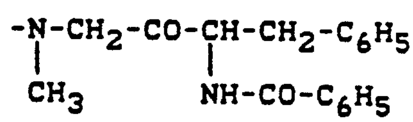 ,

$-NH-CS-NH-C_6H_5$, 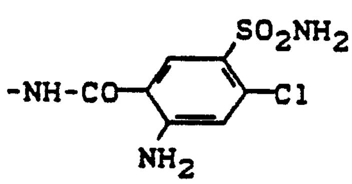 steht,

worin
t - eine Zahl 0,1,2 oder 3 bedeutet,
u - eine Zahl 0,1,2 oder 3 bedeutet,
$R^{23}$ -Wasserstoff, Alkyl, Alkylthio oder Alkylcarbonyl mit jeweils bis zu 4 Kohlenstoffatomen je Alkylgruppe, Benzoyl oder eine Gruppe der Formel

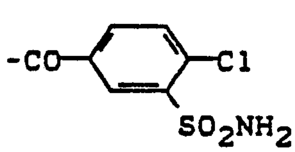 , 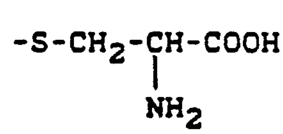 ,

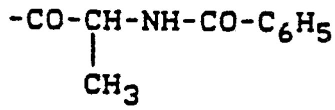 oder

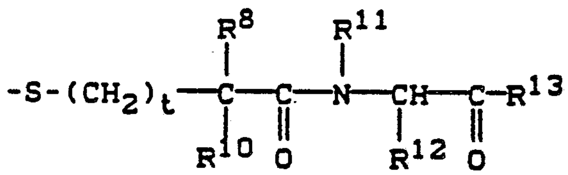 bedeutet,

$R^{24}$ -Wasserstoff, Alkyl oder Alkylcarbonyl mit jeweils bis zu 6 Kohlenstoffatomen im Alkylteil, Carboxy, Alkoxycarbonyl mit bis zu 4 Kohlenstoffatomen oder eine Gruppe der Formel

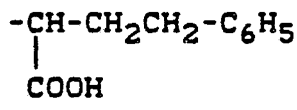 , 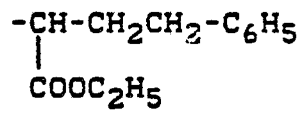 ,

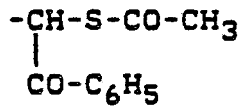 , 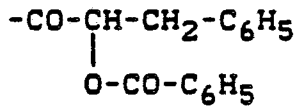

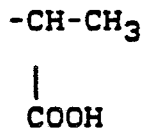 oder bedeutet 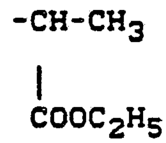

und

$R^{25}$ -Wasserstoff oder eine Gruppe der Formel

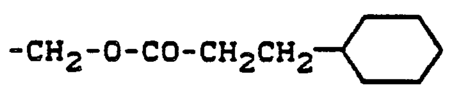 ,

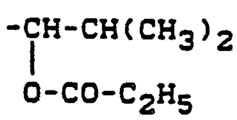 oder 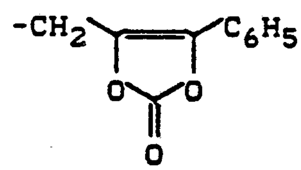

bedeutet,

$R^{10}$ -für Wasserstoff oder Alkyl mit bis zu 4Kohlenstoffatomen steht,

$R^{11}$ -für Wasserstoff oder für Alkyl it bis zu 4 Kohlenstoffatomen steht, das substituiert sein kann durch einen 5-bis 6-gliedrigen Heterocyclus mit Sauerstoff, Schwefel oder Stickstoff als Heteroatomen,oder für Cyclopentyl oder Cyclohexyl steht, das durch Phenyl substituiert sein kann,

oder

$R^{10}$ und $R^{11}$ -gemeinsam eine Gruppe der Formel

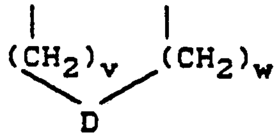

bilden,
worin
v - eine Zahl 0,1,2 oder 3 bedeutet,
w - eine Zahl 0,1 oder 2 bedeutet
und
D - eine direkte Bindung, Sauerstoff, Schwefel oder eine ortho-Phenylen-Gruppe bedeutet,
$R^{12}$ -für Wasserstoff oder für Alkyl mit bis zu 4 Kohlenstoffatomen steht, das durch eine Indolylgruppe substituiert sein kann,
oder
$R^{11}$ und $R^{12}$ -gemeinsam eine Gruppe der Formel

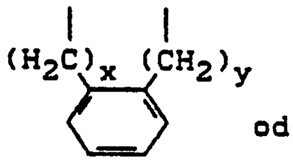

oder

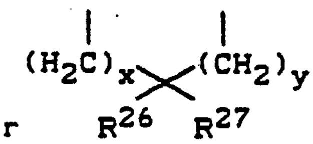

bilden,

worin
x,y - gleich oder verschieden sind und eine Zahl 0,1,2 oder 3 bedeuten,
$R^{26}$ - Wasserstoff bedeutet,
$R^{27}$ -Wasserstoff, Alkyl mit bis zu 4 Kohlenstoffatomen, Cyclopentyl, Cyclohexyl, Phenylthio, Phenylsulfonyl, Methylthio, Methylsulfonyl oder einen Rest der Formel

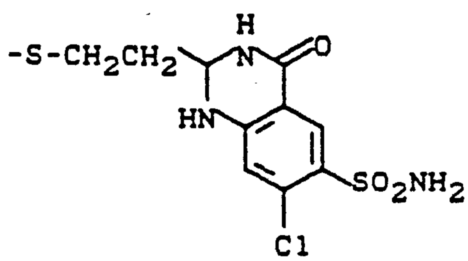

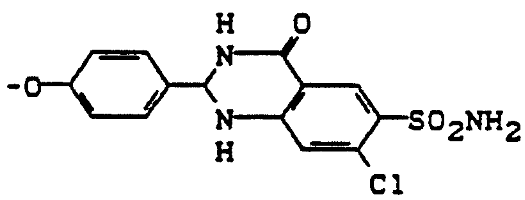

bedeutet,
oder
$R^{26}$ und $R^{27}$ gemeinsam eine Gruppe der Formel

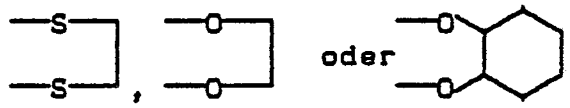 bilden

und

$R^{13}$ -für Hydroxy oder Alkoxy mit bis zu 4 Kohlenstoffatomen steht

und deren physiologisch unbedenklichen Salze, und mindestens eine Verbindung der allgemeinen Formel III (Komponente B)

in welcher

R - für Cycloalkyl mit 4 bis 12 Kohlenstoffatomen oder

-für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl, Chinoxalyl, Thionaphthenyl, Isothionaphthenyl, Chromonyl, Thiochromonyl, Chromenyl, Thiochromenyl, Benzoxadiazolyl oder Benzthiadiazolyl steht, wobei die genannten Reste substituiert sein können durch bis zu 4 gleiche oder verschiedene Substituenten der Reihe: Fluor, Chlor, Brom, Iod, Nitro, Cyano, Trifluormethyl, Monofluoralkoxy mit bis zu 8 Kohlenstoffatomen, Polyfluoralkoxy mit bis zu 8 Kohlenstoffatomen, Hydroxy, Amino, Alkylamino mit bis zu 6 Kohlenstoffatomen, Dialkylamino mit bis zu 6 Kohlenstoffatomen je Alkylgruppe, Phenyl, Naphthyl, Thienyl, Furyl, Pyridyl, Pyrimidyl, Benzyl, Benzyloxy, Benzylsulfonyl, wobei die Aromaten und Heterocyclen wiederum ein-bis vierfach substituiert sein können durch Fluor, Chlor, Brom, Iod, Cyano, Nitro, Azido, Hydroxy, Trifluormethyl, Trifluormethoxy, Amino, Alkylamino mit bis zu 6 Kohlenstoffatomen, Dialkylamino mit bis zu 6 Kohlenstoffatomen je Alkylgruppe, Alkoxy oder Alkylthio mit jeweils bis zu 2 Kohlenstoffatomen,

$R^1$ -einen geradkettigen, verzweigten oder cyclischen, gesättigten oder ungesättigten Kohlenwasserstoffrest mit bis zu 17 Kohlenstoffatomen steht, der in der Kette durch bis zu 4 Sauerstoff-und/oder Schwefelatome unterbrochen sein kann und der substituiert sein kann durch ein oder mehrere Fluor, Chlor, Brom, Iod, Nitro, Hydroxy, Cyano, Trialkylsilyl mit jeweils bis zu 6 Kohlenstoffatomen je Alkylgruppe, Alkoxycarbonyl mit bis zu 2 Kohlenstoffatomen, Amino, Alkylamino oder Dialkylamino mit jeweils bis zu 2 Kohlenstoffatomen je Alkylgruppe,

$R^2$,$R^4$ -gleich oder verschieden sind und

-für Wasserstoff, Amino, Cyano, Formyl oder

-für einen geradkettigen, verzweigten oder cyclischen, gesättigten oder ungesättigten Kohlenwasserstoffrest mit bis zu 8 Kohlenstoffatomen stehen, der durch Hydroxy, Carboxy, Alkoxycarbonyl mit bis zu 2 Kohlenstoffatomen ein oder mehreren Fluor, Chlor oder durch Brom substituiert sein kann,

$R^3$ -für Wasserstoff oder

-für einen geradkettigen, verzweigten oder cyclischen, gesättigten oder ungesättigten Kohlenwasserstoffrest mit bis zu 8 Kohlenstoffatomen steht, der durch bis zu 3 Sauerstoffatome in der Kette unterbrochen sein kann und der sub stituiert sein kann durch ein oder mehrere Fluor, Chlor, Brom, Iod, Cyano, Hydroxy, Amino, Alkylamino mit bis zu 2 Kohlenstoffatomen, Dialkylamino mit jeweils bis zu 2 Kohlenstoffatomen pro Alkylgruppe, Morpholin, Piperidin, Pyridazin oder Pyridin

und

$R^5$ -für Wasserstoff, Cyano oder Nitro steht,

oder

$R^4$ und $R^5$ -gemeinsam einen Ring bilden wie

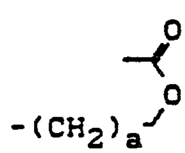 ,

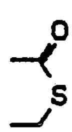 oder 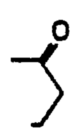 ,

wobei

a - für 1 oder 2 steht.

3. Kombinationspräparate gemäß Anspruch 1 enthaltend mindestens eine Verbindung der Komponente A der allgemeinen Formeln I und II

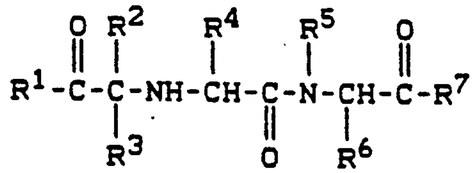 (I)

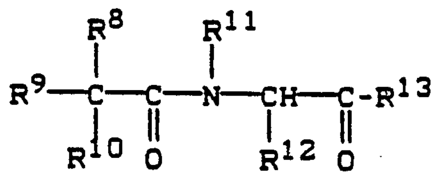 (II)

worin
$R^1$ -für Hydroxy oder Ethoxy steht,
$R^2$ -für Wasserstoff steht,
$R^3$ -für Propyl, Benzyl, 2-Phenylethyl, 2-Cyclohexylethyl, 2-(4-Piperidinyl)ethyl oder 4-Aminobutyl steht,
oder
$R^2$ und $R^3$ -gemeinsam eine Gruppe der Formel

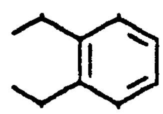

bilden,
$R^4$ -für Wasserstoff, Methyl oder 4-Aminobutyl steht,
$R^5$ -für Wasserstoff oder eine Gruppe der Formel

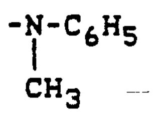 , 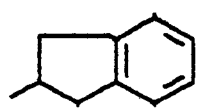 oder

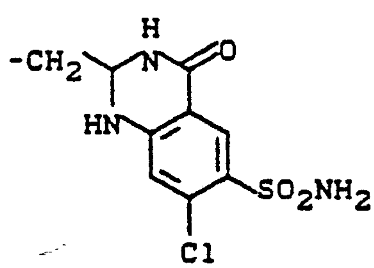 steht,

oder
$R^4$ und $R^5$ -gemeinsam eine Gruppe der Formel

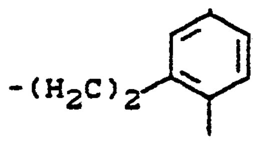, 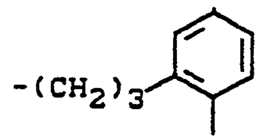, 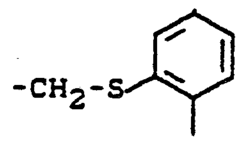

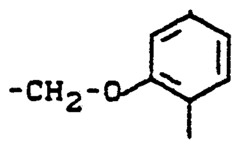 oder 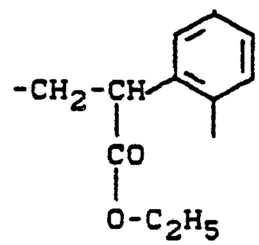 bilden,

$R^6$ -für Wasserstoff steht,
oder
$R^5$ und $R^6$ -gemeinsam eine Gruppe der Formel

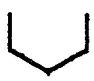, 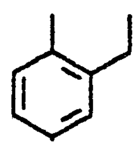, 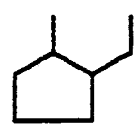, 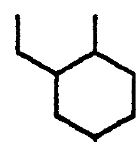,

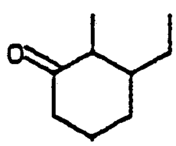, 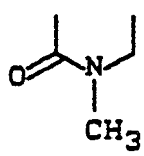, 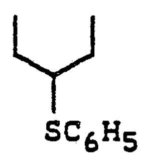, 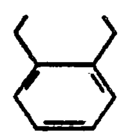 oder

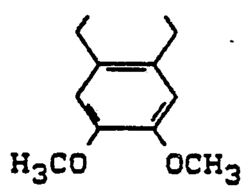 bilden

oder die Gruppierung

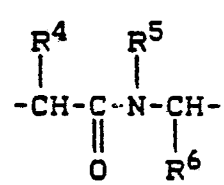

in Formel (I) für die Gruppierung der Formel

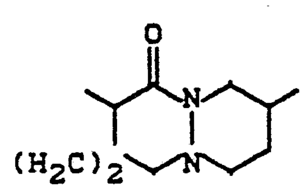

steht,
$R^7$ -für Hydroxy steht,
$R^8$ -für Wasserstoff oder Methyl steht,
$R^9$ -für eine Gruppe der Reihe

$-SH$, $-CH_2SH$, $-CH_2-S-CO-C_6H_5$, 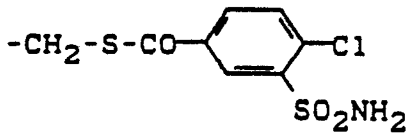

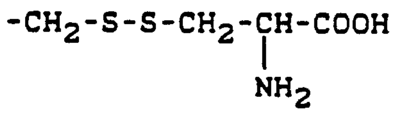 , 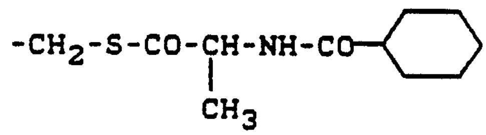

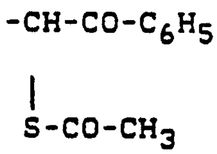 , $-CH_2-CH_2-COOH$, 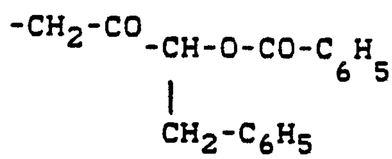

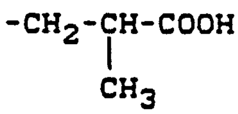 , 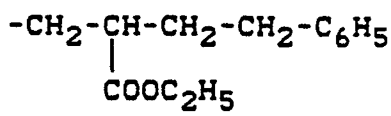

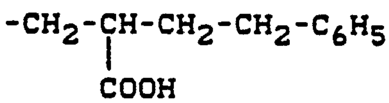 , 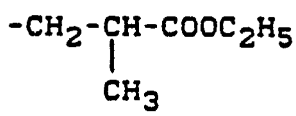

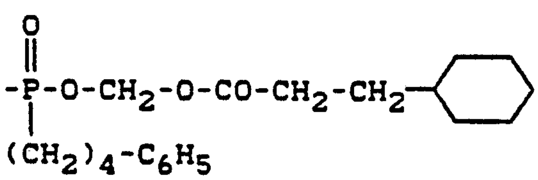 , 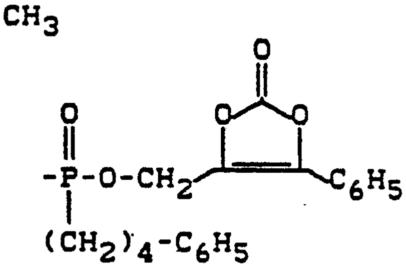

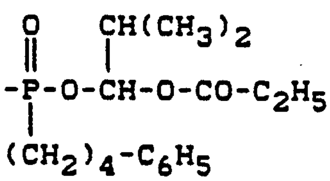 , 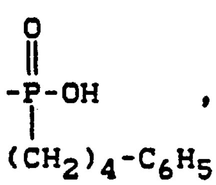 ,

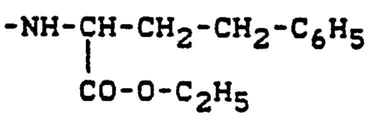 , 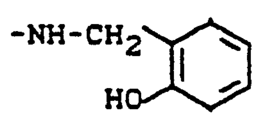 ,

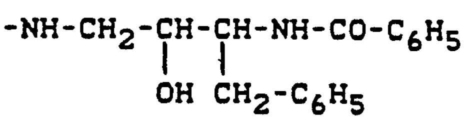 ,

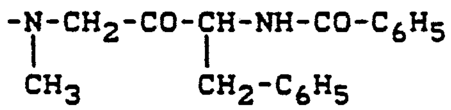 ,

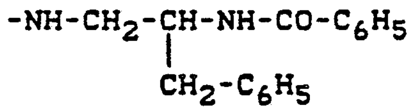 ,

$-NH-CS-NH-C_6H_5$, 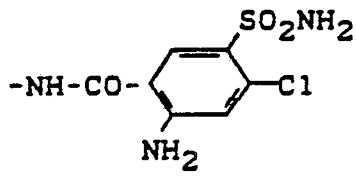 oder

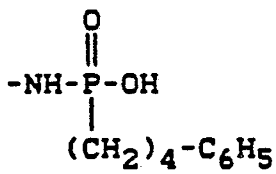 steht,

$R^{10}$ -für Wasserstoff steht,
$R^{11}$ -für Wasserstoff oder für eine Gruppe der Formel

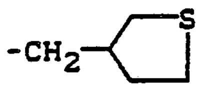 oder 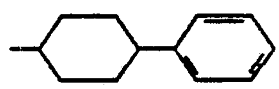 steht,

oder
$R^{10}$ und $R^{11}$ -gemeinsam eine Gruppe der Formel

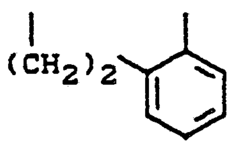 , 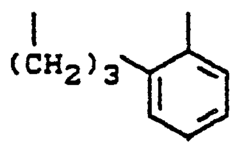 , 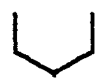 ,

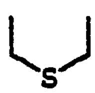 oder 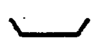 bilden,

$R^{12}$ -für Wasserstoff, Methyl oder Indol-3-ylmethyl steht,
oder
$R^{11}$ und $R^{12}$ -gemeinsam eine Gruppe der Formel

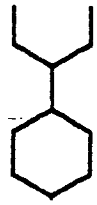 , 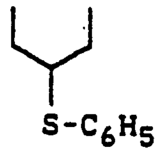 , 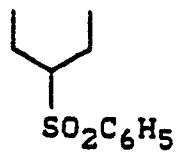 , 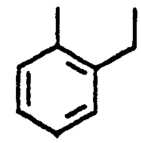 ,

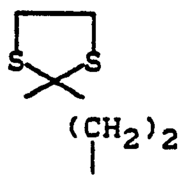 , 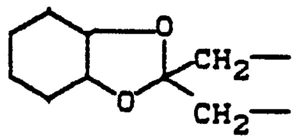

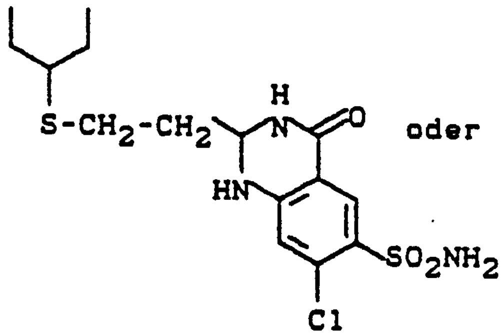 oder 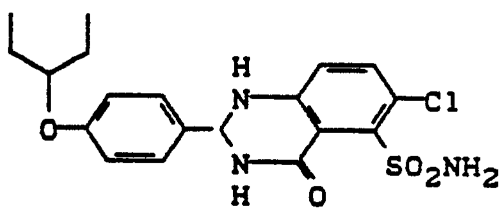

bilden

und

$R^{13}$ -für Hydroxy steht

und deren physiologisch unbedenklichen Salze und mindestens eine Verbindung der Komponente B der allgemeinen Formel III

in welcher

R - für Cycloalkyl mit 5 bis 10 Kohlenstoffatomen steht, oder

-für Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinazolyl, Chinoxalyl, Thionaphthenyl, Isothionaphthenyl, Chromonyl, Thiochromonyl, Chromenyl, Thiochromenyl, Benzoxadiazolyl oder Benzothiadiazolyl steht, wobei die genannten Reste substituiert sein können durch bis zu 3 gleiche oder verschiedene Substituenten der Reihe: Fluor, Chlor, Brom, Nitro, Cyano, Trifluormethyl, Monofluoralkoxy mit bis zu 4 Kohlenstoffatomen, Polyfluoralkoxy mit bis zu 4 Kohlenstoffatomen, Hydroxy, Amino, Alkylamino, Dialkylamino mit jeweils bis zu 4 Kohlenstoffatomen pro Alkylgruppe, Phenyl, Thienyl, Pyridyl, Benzyl, Benzyloxy oder Benzylsulfonyl, wobei die Aromaten und Heteroaromaten wiederum ein-bis dreifach substituiert sein können durch Fluor, Chlor, Brom Cyano, Nitro, Hydroxy, Trifluormethyl, Trifluormethoxy, Amino, Alkylamino mit bis zu 4 Kohlenstoffatomen, Dialkylamino mit jeweils bis zu 4 Kohlenstoffatomen je Alkylgruppe, Methoxy oder Methylthio,

$R^1$ -für einen geradkettigen, verzweigten oder cyclischen, gesättigten oder ungesättigten Kohlenwasserstoffrest mit bis zu 14 Kohlenstoffatomen steht, der durch bis zu 3 Sauerstoff-und/oder Schwefelatome in der Kette unterbrochen sein kann und der substituiert sein kann durch ein oder mehrere Fluor, Chlor, Brom, Nitro, Hydroxy, Cyano oder Trialkylsilyl mit jeweils bis zu 4 Kohlenstoffatomen je Alkylgruppe,

$R^2$,$R^4$ -gleich oder verschieden sind und

-für Wasserstoff, Amino, Cyano, Formyl oder

-für einen geradkettigen oder verzweigten, gesättigten oder ungesättigten Kohlenwasserstoffrest mit bis zu 6 Kohlenstoffatomen stehen, der durch Hydroxy substituiert sein kann,

$R^3$ -für Wasserstoff oder

-für einen geradkettigen, verzweigten oder cyclischen, gesättigten oder ungesättigten Kohlenwasserstoffrest mit bis zu 6 Kohlenstoffatomen steht, der durch bis zu 2 Sauerstoffatome in der

Kette unterbrochen sein kann und der substituiert sein kann durch ein oder mehrere Fluor, Chlor, Cyano,

Hydroxy, Amino oder Morpholino
und
$R^5$ -für Wasserstoff,
-für Cyano oder
-für Nitro steht,
oder
$R^4$ und $R^5$ -gemeinsam einen Ring bilden wie

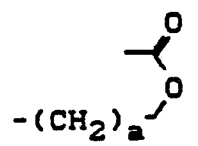 , 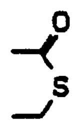 oder 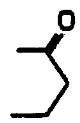 ,

wobei
a - für 1 oder 2 steht.

4. Kombinationspräparate gemäß Anspruch 1 dadurch gekennzeichnet, daß sie als Komponente B die Verbindung 1,4-Dihydro-2,6-dimethyl-5-nitro-4-(2-trifluormethylphenyl)pyridin-3-carbonsäuremethylester oder 2-Methyl-4-(4-oxo-2-phenyl-4H-thiochromen-8-yl)-5-oxo-1,4,5,7-tetrahydrofuro[3,4-b]pyridin-3-ethylester enthalten.

5. Kombinationspräparate gemäß Ansprüche 1 bis 4 dadurch gekennzeichnet, daß sie auf 1 Gew.-Tl. des ACE-Hemmers (Komponente A) 0,1 bis 10 Gew.-Tle. des positiv inotropen Dihydropyridins (Komponente B) enthalten.

6. Kombinationspräparate gemäß Anspruch 5 dadurch gekennzeichnet, daß sie auf 1 Gew.-Tl. der Komponente A 0,1 bis 5 Gew.-Tle. der Komponente B enthalten.

7. Verfahren zur Herstellung von Kombinationspräparaten enthaltend als Komponente A ACE-Hemmer der allgemeinen Formeln I und II

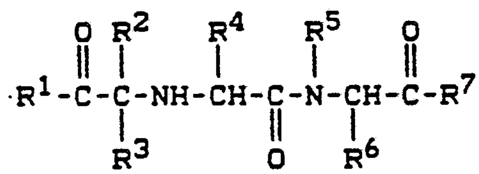 (I)

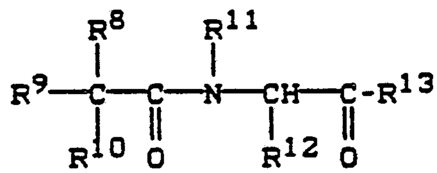 (II)

in welchen
$R^1$,$R^7$ -gleich oder verschieden sind und
-für Hydroxy, Alkoxy mit bis zu 10 Kohlenstoffatomen, Aryloxy mit 6 bis 12 Kohlenstoffatomen oder Aralkoxy mit 7 bis 14 Kohlenstoffatomen stehen,
$R^2$ -für Wasserstoff oder
-für Alkyl mit bis zu 4 Kohlenstoffatomen steht,
$R^3$ -für Wasserstoff oder
-für eine Gruppe der Formel
$-(CH_2)_m-R^{14}$ steht,
worin
m - eine Zahl 1,2,3,4,5 oder 6 bedeutet
und
$R^{14}$ -Wasserstoff, Cycloalkyl mit 3 bis 8 Kohlenstoffatomen, Hydroxy, Carboxy, Alkoxycarbonyl mit bis zu 4 Kohlenstoffatomen, Amino, Guanidino, Alkylamino mit bis zu 6 Kohlenstoffatomen, Dialkylamino mit bis zu 6 Kohlenstoffatomen je Alkylgruppe, einen 5-bis 7-gliedrigen Heterocyclus mit Sauerstoff, Schwefel oder

Stickstoff als Heteroatome, oder Aryl mit 6 bis 12 Kohlenstoffatomen bedeutet, wobei dieser Arylrest durch Halogen, Cyano, Nitro, Alkyl oder Alkoxy mit jeweils bis zu 4 Kohlenstoffatomen substituiert sein kann,

oder

$R^2$ und $R^3$gemeinsam eine Gruppe der Formel

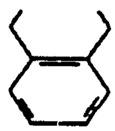

bilden,

$R^4$ -für Wasserstoff oder

-für eine Gruppe der Formel

$-(CH_2)_n-R^{15}$steht,

worin

n - die gleiche Bedeutung hat wie m und mit diesem gleich oder verschieden sein kann

und

$R^{15}$ -die gleiche Bedeutung hat wie $R^{14}$ und mit diesem gleich oder verschieden sein kann,

$R^5$ -für Wasserstoff oder

-für Alkyl mit bis zu 8 Kohlenstoffatomen steht, das substituiert sein kann durch eine Gruppe der Formel

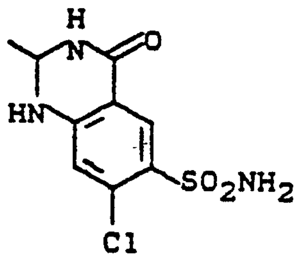

oder

-für Cycloalkyl mit 3 bis 8 Kohlenstoffatomen steht, an das ein Phenylring anelliert sein kann, oder

-für eine Gruppe $NR^{16}R^{17}$ steht,

worin

$R^{16}$,$R^{17}$ -gleich oder verschieden sind und Wasserstoff, Alkyl mit bis zu 6 Kohlenstoffatomen, Aryl mit 6 bis 12 Kohlenstoffatomen, Aralkyl mit 7 bis 14 Kohlenstoffatomen oder Acyl mit 2 bis 7 Kohlenstoffatomen bedeuten,

oder

$R^4$ und $R^5$-gemeinsam eine Gruppe der Formel

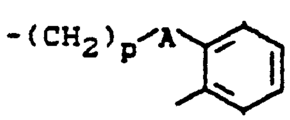

bilden,

worin

p - eine Zahl 1,2,3 oder 4 bedeutet

und

A - eine direkte Bindung, Sauerstoff, Schwefel, NH, N-Alkyl mit bis zu 6 Kohlenstoffatomen, N-Phenyl, -CHCOOH oder

-CHCOO-Alkyl mit bis zu 6 Kohlenstoffatomen in der Alkylkette bedeutet,

$R^6$ -für Wasserstoff steht ,

oder

$R^6$ und $R^5$ gemeinsam eine Gruppe der Formel

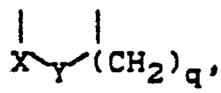, 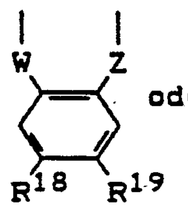 oder 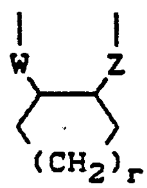 bilden,

worin

X - eine Methylen-oder Carbonylgruppe bedeutet ,

Y - Schwefel, NH, N-Alkyl mit bis zu 6 Kohlenstoffatomen in der Alkylgruppe, N-Phenyl, eine Carbonylgruppe oder eine Gruppe $-CHR^{20}$ bedeutet,

wobei

$R^{20}$ -für Hydroxy, Mercapto, Phenyloxy, Phenylthio, Alkoxy oder Alkylthio mit jeweils bis zu 6 Kohlenstoffatomen je Alkylgruppe steht,

W,Z - gleich oder verschieden sind und eine direkte Bindung oder eine Methylen-oder Ethylengruppe bedeuten,

q,r - unabhängig voneinander eine Zahl 1,2,3 oder 4 bedeuten,

$R^{18}$,$R^{19}$ gleich oder verschieden sind und Wasserstoff, Alkyl oder Alkoxy mit jeweils bis zu 6 Kohlenstoffatomen bedeuten,

oder die Gruppierung

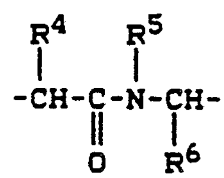

in Formel (I) für eine Gruppierung der Formel

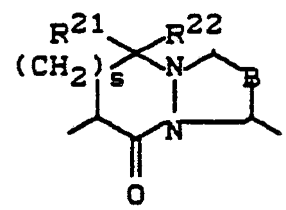

steht,

worin

s - eine Zahl 0,1,2 oder 3 bedeutet,

B - eine Methylen-, Ethylen-oder Vinylengruppe bedeutet

und

$R^{21}$,$R^{22}$ -jeweils Wasserstoff oder Alkyl mit bis zu 4 Kohlenstoffatomen oder zusammen eine Oxogruppe bedeuten,

$R^8$ -für Wasserstoff oder

-für Alkyl mit bis zu 6 Kohlenstoffatomen steht,

$R^9$ -für Wasserstoff oder

-für einen Rest der Formel

$-(CH_2)_t-S-R^{23}$, $-(CH_2)_u-R^{24}$, 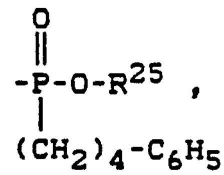 ,

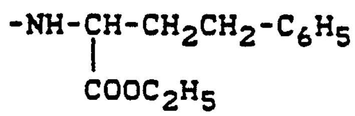 , 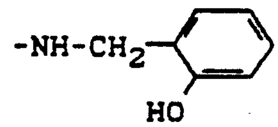 ,

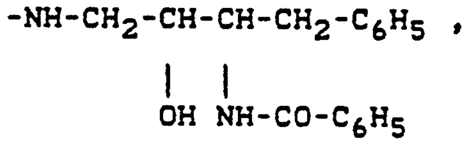 , 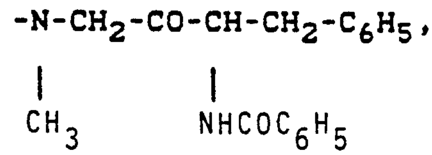 ,

$-NH-CS-NH-C_6H_5$, oder 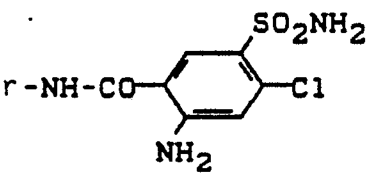 **steht,**

worin
t - eine Zahl 0,1,2,3 oder 4 bedeutet,
u - eine Zahl 0,1,2,3 oder 4 bedeutet,
$R^{23}$ -Wasserstoff, Alkyl, Alkylthio oder Alkylcarbonyl mit jeweils bis zu 6 Kohlenstoffatomen im Alkylteil, Benzoyl oder eine Gruppe der Formel

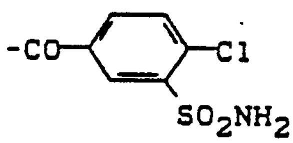 , 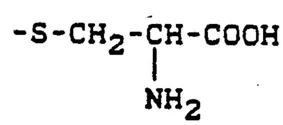 ,

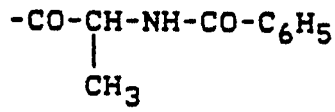 **oder**

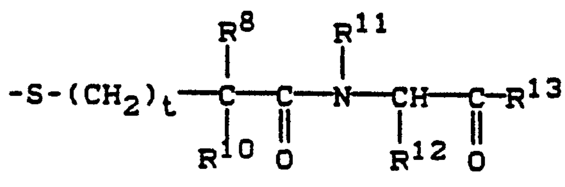 **bedeutet,**

$R^{24}$ -Wasserstoff, Alkyl oder Alkylcarbonyl mit bis zu 8 Kohlenstoffatomen im Alkylteil, Carboxy, Alkoxycarbonyl mit bis zu 6 Kohlenstoffatomen oder eine Gruppe der Formel

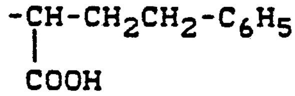 , 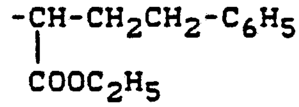 ,

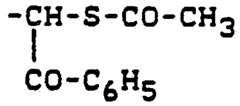 , 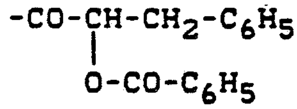

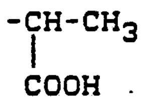 oder 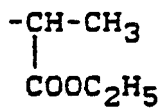 bedeutet

und

$R^{25}$ -Wasserstoff oder eine Gruppe der Formel

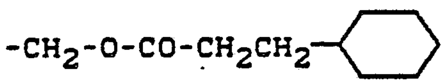 ,

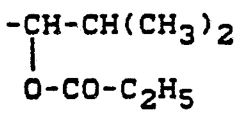 oder 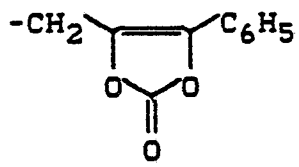

bedeutet,

$R^{10}$ -für Wasserstoff oder Alkyl mit bis zu 6 Kohlenstoffatomen steht,

$R^{11}$ -für Wasserstoff oder für Alkyl mit bis zu 8 Kohlenstoffatomen steht, das substituiert sein kann durch einen 5-bis 7-gliedrigen Heterocyclus mit Sauerstoff, Schwefel oder Stickstoff als Heteroatomen, oder -für Cycloalkyl mit 3 bis 7 Kohlenstoffatomen steht, das durch Phenyl oder Alkyl mit bis zu 4 Kohlenstoffatomen substituiert sein kann,

oder

$R^{10}$ und $R^{11}$ gemeinsam eine Gruppe der Formel

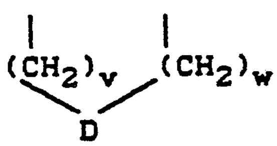

bilden,

worin

v - eine Zahl 0,1,2,3 oder 4 bedeutet,

w - eine Zahl 0,1,2 oder 3 bedeutet

und

D - eine direkte Bindung, Sauerstoff, Schwefel, NH oder eine ortho-Phenylen-Gruppe bedeutet,

$R^{12}$ -für Wasserstoff oder für Alkyl mit bis zu 6 Kohlenstoffatomen steht,das durch eine Indolylgruppe substituiert sein kann,

oder

$R^{11}$ und $R^{12}$ gemeinsam eine Gruppe der Formel

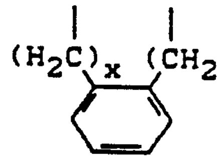 oder 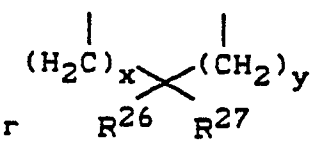 bilden ,

worin

x,y - gleich oder verschieden sind und eine Zahl 0,1,2,3 oder 4 bedeuten,

$R^{26}$ -Wasserstoff bedeutet,

$R^{27}$ -Wasserstoff, Alkyl mit bis zu 6 Kohlenstoffatomen, Cycloalkyl mit 3 bis 7 Kohlenstoffatomen, Phenylthio, Phenylsulfonyl, Alkylthio oder Alkylsulfonyl mit jeweils bis zu 4 Kohlenstoffatomen, oder einen Rest der Formel

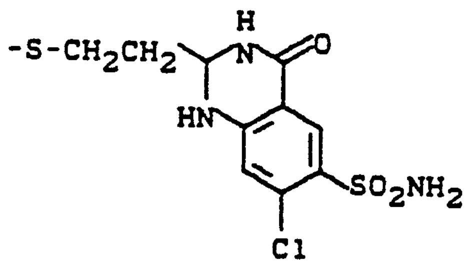 oder

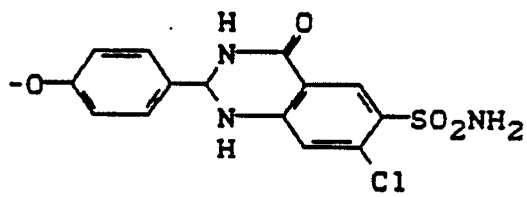

bedeutet,

oder

$R^{26}$ und $R^{27}$ gemeinsam eine Gruppe der Formel

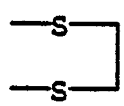 , 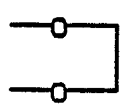 oder 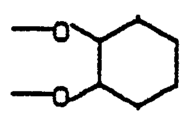 bilden

und

$R^{13}$ -für Hydroxy oder Alkoxy mit bis zu 6 Kohlenstoffatomen steht,

und deren physiologisch unbedenklichen Salze, sowie positivinotrop wirkende Dihdyropyridinverbindungen (Komponente B) der allgemeinen Formel III

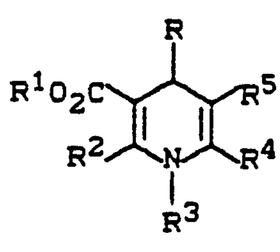 (III)

in welcher

R - für Cycloalkyl mit 3 bis 14 Kohlenstoffatomen,
-für Aryl mit 6 bis 14 Kohlenstoffatomen oder für Heteroaryl steht, die substituiert sein können durch bis 5 gleiche oder verschiedene Substituenten der Reihe: Halogen, Nitro, Cyano, Trifluormethyl, Monofluoralkoxy mit bis zu 12 Kohlenstoffatomen, Polyfluoralkoxy mit bis zu 12 Kohlenstoffatomen, Hydroxy, Amino, Alkylamino mit bis zu 8 Kohlenstoffatomen, Dialkylamino mit bis zu 8 Kohlenstoffatomen je Alkylgruppe, Aryl mit 6 bis 12 Kohlenstoffatomen, Heteroaryl, Aralkyl mit 7 bis 14 Kohlenstoffatomen, Aralkoxy mit 7 bis 14 Kohlenstoffatomen oder -$SO_n$ -Aralkyl mit 7 bis 14 Kohlenstoffatomen und n = 0 bis 2, wobei die Substituenten der fünf letzt genannten Gruppen ebenfalls mehrfach substituiert sein können durch bis zu 5 Substituenten der Reihe: Halogen, Nitro, Azido, Hydroxy, Trifluormethyl, Trifluormethoxy, Cyano, Amino, Alkylamino mit bis zu 8 Kohlenstoffatomen, Dialkylamino mit jeweils bis zu 8 Kohlenstoffatomen je Alkylgruppe, Alkoxy oder Alkylthio mit jeweils bis zu 4 Kohlenstoffatomen,
$R^1$ -für einen geradkettigen, verzweigten oder cyclischen, gesättigten oder ungesättigten Kohlenwasserstoffrest mit bis zu 20 Kohlenstoffatomen steht, der bis zu 5 Schwefel und/oder Sauerstoffatome in der Kette unterbrochen sein kann und der substituiert sein kann durch Halogen, Nitro, Hydroxy, Cyano, Trialkylsilyl, mit jeweils bis zu 8 Kohlenstoffatomen pro Alkylgruppe, Alkoxycarbonyl mit bis zu 4 Kohlenstoffatomen, Amino, Alkylamino mit bis zu 4 Kohlenstoffatomen oder Dialkylamino mit jeweils bis zu 4 Kohlenstoffatomen je Alkylgruppe,
$R^2$,$R^4$ -gleich oder verschieden sind und
-für Wasserstoff, Amino, Cyano, Formyl oder
-für einen geradkettigen, verzweigten oder cyclischen, gesättigten oder ungesättigten Kohlenwasserstoffrest mit bis zu 10 Kohlenstoffatomen stehen, der substituiert sein kann durch Hydroxy, Carboxy, Alkoxycarbonyl mit bis zu 4 Kohlenstoffatomen oder durch Halogen,
$R^3$ -für Wasserstoff oder
-für einen geradkettigen, verzweigten oder cyclischen, gesättigten oder ungesättigten Kohlenwasserstoffrest mit bis zu 10 Kohlenstoffatomen steht, der substituiert sein kann durch Halogen, Cyano, Hydroxy, Amino, Alkylamino, Dialkylamino mit jeweils bis zu 4 Kohlenstoffatomen je Alkylgruppe oder durch einen 5-bis 7-gliedrigen heterocyclischen Ring, der als Heteroatome Stickstoff und/oder Sauerstoff und/oder Schwefel enthalten kann und sowohl gesättigt als auch ungesättigt sein kann
und
$R^5$ -für Wasserstoff,
-für Cyano oder
-für Nitro steht,
oder
$R^4$ und $R^5$ -gemeinsam einen Ring bilden wie

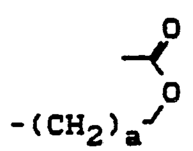 , 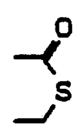 oder 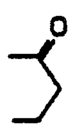

wobei
a - für 1 oder 2 steht,
dadurch gekennzeichnet, daß man die Komponenten A und B in einem Einzelkomponenten auflösenden inerten Lösungsmittel auflöst und anschließend gegebenenfalls nach Abdampfen des Lösungsmittels mit Hilfsstoffen vemischt und in eine geeignete Applikationsform überführt.

8. Verfahren gemäß Anspruch 7 dadurch gekennzeichnet, daß man als Lösungsmittel niederer Alkohol wie Ethanol oder flüssige Glykole wie Polyethylenglykol verwendet.

9. Verwendung von Kombinationspräparaten gemäß Anspruch 1 bei der Bekämpfung von Herz-und Kreislauferkrankungen.

10. Verwendung von Kombinationspräparaten gemäß Anspruch 1 bei der Behandlung von Herzinsuffizienz.